(19) Europäisches Patentamt / European Patent Office / Office européen des brevets

(11) **EP 3 030 093 B1**

(12) **EUROPEAN PATENT SPECIFICATION**

(45) Date of publication and mention of the grant of the patent:
**06.09.2017   Bulletin 2017/36**

(21) Application number: **14744322.0**

(22) Date of filing: **25.07.2014**

(51) Int Cl.:
*A23K 10/16* (2016.01)

(86) International application number:
**PCT/EP2014/066078**

(87) International publication number:
**WO 2015/011276 (29.01.2015 Gazette 2015/04)**

(54) **POLYPEPTIDES HAVING alpha-L-GALACTOSIDASE ACTIVITY AND POLYNUCLEOTIDES ENCODING SAME**

POLYPEPTIDE MIT alpha-L-GALACTOSIDASE-AKTIVITÄT UND POLYNUKLEOTIDE ZUR CODIERUNG DAVON

POLYPEPTIDES PRÉSENTANT UNE ACTIVITÉ alpha-L-GALACTOSIDASE ET POLYNUCLÉOTIDES CODANT POUR CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **26.07.2013   EP 13178257**

(43) Date of publication of application:
**15.06.2016   Bulletin 2016/24**

(73) Proprietor: **Novozymes A/S**
**2880 Bagsvaerd (DK)**

(72) Inventors:
- **KROGH, Kristian, Bertel, Rømer, M.**
  **DK-2880 Bagsværd (DK)**
- **EKLÖF, Jens Magnus**
  **DK-2100 Copenhagen Ø (DK)**
- **JOHANSEN, Katja, Salomon**
  **DK-2820 Gentofte (DK)**
- **ROGOWSKI, Artur**
  **Newcastle upon Tyne**
  **Northumberland NE2 1EZ (GB)**
- **BOLAM, David, N.**
  **Sunderland**
  **Tyne and Wear SR3 3PJ (GB)**
- **GILBERT, Harry, J.**
  **Ovington**
  **Northumberland NE42 6EB (GB)**

(56) References cited:
**WO-A1-94/23022          WO-A1-2005/003329**
**US-A1- 2005 100 892**

- **PAN BAOHAI ET AL: "Effect of dietary supplementation with alpha-galactosidase preparation and stachyose on growth performance, nutrient digestibility and intestinal bacterial populations of piglets", ARCHIV FUER TIERERNAEHRUNG - ARCHIVES OF ANIMAL NUTRITION, AKADEMIE VERLAG, BERLIN, DE, vol. 56, no. 5, 1 October 2002 (2002-10-01), pages 327-337, XP009180754, ISSN: 0003-942X**
- **FROIDMONT ERIC ET AL: "Improvement of lupin seed valorisation by the pig with the addition of alpha-galactosidase in the feed and the choice of a suited variety", BIOTECHNOLOGIE, AGRONOMIE, SOCIETE ET ENVIRONMENT,, vol. 9, no. 4, 1 January 2005 (2005-01-01) , pages 225-235, XP009180753, ISSN: 1370-6233**
- **A. VELDMAN ET AL: "Effect of [alpha]-galactosides and [alpha]-galactosidase in feed on ileal piglet digestive physiology", JOURNAL OF ANIMAL PHYSIOLOGY AND ANIMAL NUTRITION, vol. 69, no. 1-5, 8 January 1993 (1993-01-08), pages 57-65, XP55146740, ISSN: 0931-2439, DOI: 10.1111/j.1439-0396.1993.tb00790.x**
- **DU FANG ET AL: "Purification and characterization of an [alpha]-galactosidase fromPhaseolus coccineusseeds showing degrading capability on raffinose family oligosacchar", PLANT PHYSIOLOGY AND BIOCHEMISTRY, GAUTHIER-VILLARS, PARIS, FR, vol. 69, 9 May 2013 (2013-05-09), pages 49-53, XP028565895, ISSN: 0981-9428, DOI: 10.1016/J.PLAPHY.2013.04.017**

**(Cont. next page)**

• GHAZI S ET AL: "Improvement of the nutritive value of soybean meal by protease and alpha-galactosidase treatment in broiler cockerels and broiler chicks", BRITISH POULTRY SCIENCE, LONGMAN GROUP, GB, vol. 44, no. 3, 1 July 2003 (2003-07-01), pages 410-418, XP009180755, ISSN: 0007-1668

• ETIENNE REBUFFET ET AL: "Discovery and structural characterization of a novel glycosidase family of marine origin", ENVIRONMENTAL MICROBIOLOGY, vol. 13, no. 5, 18 February 2011 (2011-02-18), pages 1253-1270, XP55147079, ISSN: 1462-2912, DOI: 10.1111/j.1462-2920.2011.02426.x

## Description

### Reference to a Sequence Listing

This application contains a Sequence Listing in computer readable form.

### Reference to a Deposit of Biological Material

This application contains a reference to a public deposit of biological material, (ATCC 8483).

## Background of the Invention

### Field of the Invention

The present invention relates to polypeptides having $\alpha$-L-galactosidase activity and a catalytic domain belonging to Glycoside Hydrolase family 95 (GH95, www.cazy.org PMID:18838391). Also disclosed are nucleic acid constructs, vectors, and host cells comprising the polynucleotides as well as methods of producing and using the polypeptides and catalytic domain.

### Description of the Related Art

The presence of $\alpha$-l-galactosyl sugar moieties in corn xylan has been described in literature (Allerdings et al. 2006, Phytochemistry 67:1276-1286) but an enzyme responsible for the release of this sugar moiety has not been described previously. The same sugar moiety can also be found on jojoba seed xyloglucan (Hantus et al. 1997, Carbohydr. Res. 304:11-20) and in xyloglucan and rhamnogalacturonan II from Arabidopsis mur1 mutants (Zablackis et al. 1996, Science 272:1808-1810, Reuhs et al. 2004, Planta 219:147-157). The present invention provides polypeptides from GH95 having $\alpha$-L-galactosidase activity and polynucleotides encoding the polypeptides, especially the GH95 $\alpha$-L-galactosidase designated BACOVA 03438.

A number of sequences have been published in the UNIPROT database (reference) that are indicated as as Glycosyl hydrolases belonging to family 31 (EC=3.2.1.-;) or simply as "protein". This includes also SEQ ID NOs: 2 and 3 as exemplified herein. The sequence identities of these sequences to SEQ ID NO:2 range from 100% to 97.8%. Further related sequences (by sequence identity) are below 77.2% identity.

SEQ ID NO: 2 is thus published as UNIPROT: A7M011 with the reference Fulton,L Clifton,S Fulton,B Xu,J Minx,P Pepin,KH Johnson,M Thiruvilangam,P Bhonagiri,V Nash,WE Mardis,ER Wilson,RK. Submitted (MAR-2007) to the EMBL/GenBank/DDBJ databases, and Sudarsanam,P Ley,R Guruge,J Turnbaugh,PJ Mahowald,M Liep,D Gordon,J. Draft genome sequence of *Bacteroides ovatus* (ATCC 8483). Submitted (APR-2007) to the EMBL/GenBank/DDBJ databases.

Sequences having between 100 and 92.6% identities to SEQ ID NO:2 are also disclosed, e.g. UNIPROT: I8Z241 (100% Bacteroides ovatus CL03T12C18), UNIPROT:I8YAG0 (99.9% Bacteroides ovatus CL02T12C04), UNIPROT:I9US19 (98.9% Bacteroides xylanisolvens CL03T12C04), UNIPROT:D0TM59 (Bacteroides sp. strain 2_1_22), and some more.

### Summary of the Invention

The present invention relates to the use in animal feed of isolated polypeptides having $\alpha$-L-galactosidase activity selected from the group consisting of:

(a) a polypeptide having at least 80% sequence identity to the mature polypeptide of SEQ ID NO: 2;
(b) a polypeptide encoded by a polynucleotide that hybridizes under high, or very high stringency conditions with (i) the mature polypeptide coding sequence of SEQ ID NO: 1 or (ii) the full-length complement of (i);
(c) a polypeptide encoded by a polynucleotide having at least 92% sequence identity to the mature polypeptide coding sequence of SEQ ID NO: 1
(d) a variant of the mature polypeptide of SEQ ID NO: 2 comprising a substitution, deletion, and/or insertion at one or more (e.g., several) positions; and
(e) a fragment of the polypeptide of (a), (b), (c), or (d) that has $\alpha$-L-galactosidase activity.

The present invention also relates to isolated polypeptides comprising a catalytic domain selected from the group consisting of:

(a) a catalytic domain having at least 80% sequence identity to amino acids 20 to 811 of SEQ ID NO: 2

(b) a catalytic domain encoded by a polynucleotide that hybridizes under high, or very high stringency conditions with (i) nucleotides 60 to 2436 of SEQ ID NO: 1, or (iii) the full-length complement of (i) or (ii);

(c) a catalytic domain encoded by a polynucleotide having at least 92% sequence identity to nucleotides 60 to 2436 of SEQ ID NO: 1

(d) a variant of amino acids 20 to 811 of SEQ ID NO: 2 comprising a substitution, deletion, and/or insertion at one or more (e.g., several) positions; and

(e) a fragment of the catalytic domain of (a), (b), (c), or (d) that has α-L-galactosidase activity.

The present invention also relates to isolated polynucleotides encoding the polypeptides of the present invention; nucleic acid constructs; recombinant expression vectors; recombinant host cells comprising the polynucleotides; and methods of producing the polypeptides.

The present invention also relates to variant polypeptides having α-L-galactosidase activity and having at least 80%, e.g. at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to SEQ ID NO: 3 comprising at least one substitution, deletion, and/or insertion of at least one or more (several) amino acids of SEQ ID NO: 3.

The present invention further relates to compositions comprising an isolated polypeptide having α-L-galactosidase activity, selected from the group consisting of:

(a) a polypeptide having at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 3;

(b) a polypeptide encoded by a polynucleotide that hybridizes under, high stringency conditions or very-high stringency conditions with:

(i) the mature polypeptide coding sequence of SEQ ID NO: 1; and/or
(ii) the full-length complementary strand of (i);

(c) a polypeptide encoded by a polynucleotide having at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 1;

(d) a variant comprising a substitution, deletion, and/or insertion of one or more (several) amino acids of SEQ ID NO: 3; and

(e) a fragment of a polypeptide of (a), (b), (c) or (d), that has α-L-galactosidase activity.

The compositions can be animal feed compositions. The present invention also relates to isolated polynucleotides encoding the polypeptides of the present invention, nucleic acid constructs, recombinant expression vectors, recombinant host cells comprising the polynucleotides, and to methods of recombinantly producing the polypeptides. The present invention also relates to methods for preparing a composition for use in animal feed, methods for improving the nutritional value of an animal feed, and methods of treating proteins to be used in animal feed compositions. Also disclosed herein is a polynucleotide encoding a signal peptide comprising or consisting of amino acids 1 to 20 of SEQ ID NO: 2, a polynucleotide encoding a propeptide comprising or consisting of amino acids 1 to 20 of SEQ ID NO: 2, or a polynucleotide encoding a signal peptide and a propeptide comprising or consisting of amino acids 1 to 20 of SEQ ID NO: 2, each of which is operably linked to a gene encoding a protein; nucleic acid constructs, expression vectors, and recombinant host cells comprising the polynucleotides; and methods of producing a protein used in the invention.

**Brief Description of the Figures**

Fig. 1 shows that the enzyme of this invention does not hydrolyse any of the commercially available arabinoxylans og glucuronoxylans tested. The figure shows thin layer chromatography indicating activity *B.ovatus* GH95 (BACOVA_03438) enzyme of the invention on corn bran xylan. *B.ovatus* GH95 releases single product from corn xylan (lane C). Lane (A) X1 to X5 mix of xylooligosaccharide standards. Lane (B) untreated corn bran xylan.

Fig. 2 shows High performance liquid chromatography (HPLC) showing that the enzyme of this invention, GH95 (BACOVA_03438), releases L-galactose and not fucose from corn bran xylan.

Fig. 3 shows that the *B.ovatus* GH95 enzyme of the invention is -1000 times more active on corn xylan than *B.bifidum* GH95.

Fig. 4 shows the difference between relative rates of the GH95 (BACOVA_03438) and *B.bifidum* GH95 (AAQ72464) against corn bran xylan and 2' -fucosyllactose. *B. ovatus* GH95 $\alpha$-L-galactosidase is at least 200x more active on corn xylan than *B.bifidum* GH95 $\alpha$-fucosidase (panel A). Relative rate of the two enzymes against 2-fucosyllactose (Panel B). *B. ovatus* GH95 $\alpha$-L-galactosidase is at least 5000x less active on 2'-fucosyllactose than *B.bifidum* $\alpha$-L-fucosidase.

Fig. 5 shows monosaccharides released from corn fiber gum after three different enzyme treatments. The enzymes were the GH95 $\alpha$-L-galactosidase, UIrFlo L (from Novozymes A/S) and a combination thereof. The monosaccharides are as follows: arabinose, galactose, glucose, xylose.

Fig. 6 shows a further combination with the additional B. *ovatus* GH31 $\alpha$-xylosidase (disclosed in our concurrently filed patent application).

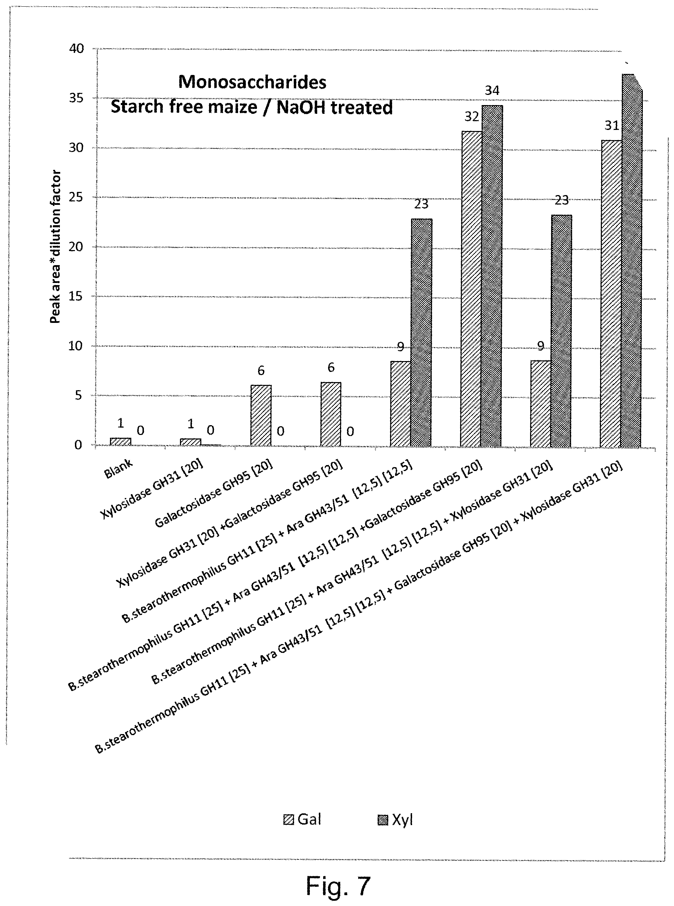

Fig. 7 shows the effect of adding the B. *ovatus* GH95 enzyme together with a mixture of individual hemicellulases on the release of monosaccharides from destarched corn bran. Sugar levels are expressed as area under curve times a dilution factor. The figure also shows the effects of GH95 $\alpha$-L-galactosidase and other enzymes (GH11, GH43/51 and combinations.

## Brief Desciption of the Sequence Listing

In the sequence listing

SEQ ID NO: 1 is the genomic/cDNA sequence of the GH95 a-L-galactosidase from *Bacteroides ovatus* ATCC 8483, including the start codon to the stop codon without introns.

SEQ ID NO: 2 is the protein sequence deducted from SEQ ID NO:1 (same as UNIPROT:A7M011).

SEQ ID NO: 3 is the amino acid sequence from Bacteroides ovatus CL03T12C18 (UNIPROT:I8Z241, same as UNIPROT:A7M011)

SEQ ID NO: 4 is the amino acid sequence from Bacteroides ovatus CL02T12C04 (UNIPROT:I8YAG0)

SEQ ID NO: 5 is the amino acid sequence from Bacteroides xylanisolvens CL03T12C04 (UNIPROT:I9US19)

SEQ ID NO: 6 is the amino acid sequence from Bacteroides sp. strain 2_1_22 (UNIPROT:D0TM59)

**Identity Matrix of sequences:**

| SEQ ID NO: | 2 | 3 | 4 | 5 | 6 |
|---|---|---|---|---|---|
| 2 | 100 | 100 | 99.9 | 98.9 | 92.2 |
| 3 | 100 | 100 | 98.2 | 99.0 | 99.2 |
| 4 | 99.9 | 99.9 | 100 | 98.6 | 92.1 |
| 5 | 98.9 | 98.9 | 98.8 | 100 | 92.6 |
| 6 | 92.2 | 92.2 | 92.1 | 92.6 | 100 |

## Definitions

**$\alpha$-L-galactosidase:** The term "$\alpha$-L-galactosidase" means an enzyme activity that catalyses the hydrolysis of an $\alpha$-L-galactosyl unit from a substrate releasing L-galactose (No EC number has yet been established for this activity). For purposes of the present invention, $\alpha$-L-galactosidase activity is determined according to the procedure described in the Examples. In one aspect, the polypeptides of the present invention have at least 20%, e.g., at least 40%, at least 50%, at least 60%, at least 70%, at least 80%, at least 90%, at least 95%, or at least 100% of the $\alpha$-L-galactosidase activity of the mature polypeptide of SEQ ID NO: 2.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native (*i.e.,* from the same gene) or foreign (*i.e.,* from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Fragment:** The term "fragment" means a polypeptide or a catalytic domain having one or more (e.g., several) amino acids absent from the amino and/or carboxyl terminus of a mature polypeptide or domain; wherein the fragment has $\alpha$-L-galactosidase activity. In one aspect, a fragment contains at least 365 amino acid residues (e.g., amino acids 321 to 686 of SEQ ID NO: 2), at least 666 amino acid residues (e.g., amino acids 20 to 686 of SEQ ID NO: 2), or at least 760 amino acid residues (e.g., amino acids 20 to 780 of SEQ ID NO: 2). In one aspect, a fragment contains at least at least 85%, at least 90% or at least 95% of the amino acids of the mature peptide.

**Glycoside hydrolases (EC 3.2.1.-):** are a widespread group of enzymes which hydrolyse the glycosidic bond between two or more carbohydrates or between a carbohydrate and a non-carbohydrate moiety. The IUBMB Enzyme nomenclature of glycoside hydrolases is based on their substrate specificity and occasionally on their molecular mechanism; such a classification does not reflect (and was not intended to) the structural features of these enzymes. A classification of glycoside hydrolases in families based on amino acid sequence similarities has been proposed a few years ago (CAZy). Because there is a direct relationship between sequence and folding similarities, this classification:

(i) reflects the structural features of these enzymes better than their sole substrate specificity,

(ii) helps to reveal the evolutionary relationships between these enzymes,

(iii) provides a convenient tool to derive mechanistic information, and

(iv) illustrates the difficulty of deriving relationships between family membership and substrate specificity

The Carbohydrate-Active Enzymes database (**CAZy**) provides a continuously updated list of the glycoside hydrolase families. Because the fold of proteins is better conserved than their sequences, some of the families can be grouped in '**clans**' :

(i) when new sequences are found to be related to more than one family,

(ii) when the sensitivity of sequence comparison methods is increased or

(iii) when structural determinations demonstrate the resemblance between members of different families [3]

**The GH95 enzymes:** are known to exhibit $\alpha$-1,2-L-fucosidase (EC 3.2.1.63); and/or $\alpha$ -L-fucosidase (EC 3.2.1.51) activity.

**Stringency conditions**

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

**Low stringency conditions:** The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

**Host cell:** The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated *(e.g.,* recombinant production in a host cell; multiple copies of a gene encoding the substance; and use of a stronger promoter than the promoter naturally associated with the gene encoding the substance).

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 20 to 811 of SEQ ID NO: 2 based on the SignalP program (Nielsen et al., 1997, Protein Engineering 10: 1-6) that indicates amino acids 1 to 20 of SEQ ID NO: 2 are a signal peptide. It is known in the art that a host cell may produce a mixture of two of more different mature polypeptides *(i.e.,* with a different C-terminal and/or N-terminal amino acid) expressed by the same polynucleotide. It is also known in the art that different host cells process polypeptides differently, and thus, one host cell expressing a polynucleotide may produce a different mature polypeptide (e.g., having a different C-terminal and/or N-terminal amino acid) as compared to another host cell expressing the same polynucleotide. In one aspect, a mature polypeptide contains up to 792 amino acid residues (e.g., amino acids 1 to 792 of SEQ ID NO: 2), up to 780 amino acid residues (e.g., amino acids 10 to 772 of SEQ ID NO: 2),

**Mature polypeptide coding sequence:** The term "mature polypeptide coding sequence" means a polynucleotide that encodes a mature polypeptide having $\alpha$-L-galactosidase activity. In one aspect, the mature polypeptide coding sequence is nucleotides 60 to 2433 of SEQ ID NO:1 as found by SignalP (Nielsen *et al.,* 1997, *supra)* that indicates nucleotides 1 to 60 of SEQ ID NO: 1 encode a signal peptide.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$\text{(Identical Residues} \times 100)/(\text{Length of Alignment} - \text{Total Number of Gaps in Alignment})$$

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice et al., 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Deoxyribonucleotides} \times 100)/(\text{Length of Alignment} - \text{Total Number of Gaps in Alignment})$$

**Subsequence:** The term "subsequence" means a polynucleotide having one or more *(e.g.,* several) nucleotides absent from the 5' and/or 3' end of a mature polypeptide coding sequence; wherein the subsequence encodes a fragment having α-L-galactosidase activity. In one aspect, a subsequence contains at least 1095 nucleotides (e.g., nucleotides 963 to 2058 of SEQ ID NO: 1), at least 1998 nucleotides (e.g., nucleotides 60 to 2058 of SEQ ID NO: 1), or at least 2280 nucleotides (e.g., nucleotides 60 to 2340 of SEQ ID NO: 1). In one aspect, a subsequence contains at least 85%, 90% or 95% of the nucleotides coding the mature polypeptide of SEQ ID NO:2

**Variant:** The term "variant" means a polypeptide having α-L-galactosidase activity comprising an alteration, *i.e.,* a substitution, insertion, and/or deletion, at one or more (e.g., several) positions. A substitution means replacement of the amino acid occupying a position with a different amino acid; a deletion means removal of one or more (e.g., several) amino acids, e.g., 1-5 amino acids, adjacent to the amino acid occupying a position; and an insertion means adding one or more (e.g., several) amino acids, e.g., 1-5 amino acids, adjacent to the amino acid occupying a position.

**Detailed Description of the Invention**

**Polypeptides Having α-L-Galactosidase Activity**

In an embodiment, the present invention relates to the use in animal feed of isolated polypeptides having a sequence identity to the mature polypeptide of SEQ ID NO: 2 of at least 80%, at least 82%, at least 84%, at least 85%, at least 87%, at least 89%, at least 90%, at least 92%, at least 93%, at least 94%, at least 95%, at least 96%, at least 97%, at least 98%, at least 99%, or 100%, which have α-L-galactosidase activity. In one aspect, the polypeptides differ by up to to 30, 25, 20, 19, 18, 17, 16, 15, 14, 13, 12, 11, or 10 amino acids, e.g., 1, 2, 3, 4, 5, 6, 7, 8, 9, or 10, from the mature polypeptide of SEQ ID NO: 2.

A polypeptide of the present invention preferably comprises or consists of the amino acid sequence of SEQ ID NO: 2 or an allelic variant thereof; or is a fragment thereof having α-L-galactosidase activity. In another aspect, the polypeptide comprises or consists of the mature polypeptide of SEQ ID NO: 2. In another aspect, the polypeptide comprises or consists of amino acids 25 to 750 of SEQ ID NO: 2.

The present invention also relates to the use in animal feed of isolated polypeptides having a sequence identity to the mature polypeptide of SEQ ID NO: 4 of at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%, which have α-L-galactosidase activity. In one aspect, the polypeptides differ by no than thirty-six amino acids, e.g., by thirty amino acids, by twenty-five amino acids, by twenty amino acids, by fifteen amino acids, by ten amino acids, by nine amino acids, by eight amino acids, by seven amino acids, by six amino acids, by five amino acids, by four amino acids, by three amino acids, by two amino acids, and by one amino acid from the mature polypeptide of SEQ ID NO: 4.

The present invention further relates to the use in animal feed of isolated polypeptides having a sequence identity to the mature polypeptide of SEQ ID NO: 5 of at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%, which have α-L-galactosidase activity. In one aspect, the polypeptides differ by no more than thirty-six amino acids, e.g., by thirty amino acids, by twenty-five amino acids, by twenty amino acids, by fifteen amino acids, by ten amino acids, by nine amino acids, by eight amino acids, by seven amino acids, by six amino acids, by five amino acids, by four amino acids, by three amino acids, by two amino acids, and by one amino acid from the mature polypeptide of SEQ ID NO: 5.

The present invention further relates to the use in animal feed of isolated polypeptides having a sequence identity to the mature polypeptide of SEQ ID NO: 6 of at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%, which have α-L-galactosidase activity. In one aspect, the polypeptides differ by no more than thirty-six amino acids, e.g., by thirty amino acids, by twenty-five amino acids, by twenty amino acids, by fifteen amino acids, by ten amino acids, by nine amino acids, by eight amino acids, by seven amino acids, by six amino acids, by five amino acids, by four amino acids, by three amino acids, by two amino acids, and by one amino acid from the mature polypeptide of SEQ ID NO: 6.

An embodiment of the invention uses a polypeptide or a polypeptide encoded by a polynucleotide in animal feed having at least 80% sequence identity to the polypeptide of SEQ ID NO: 2.

An embodiment of the invention uses a polypeptide or a polypeptide encoded by a polynucleotide in animal feed having at least 82% sequence identity to the polypeptide of SEQ ID NO: 2.

An embodiment of the invention uses a polypeptide or a polypeptide encoded by a polynucleotide in animal feed having at least 84% sequence identity to the polypeptide of SEQ ID NO: 2.

An embodiment of the invention uses a polypeptide or a polypeptide encoded by a polynucleotide in animal feed having at least 85% sequence identity to the polypeptide of SEQ ID NO: 2.

An embodiment of the invention uses a polypeptide or a polypeptide encoded by a polynucleotide in animal feed having at least 86% sequence identity to the polypeptide of SEQ ID NO: 2.

An embodiment of the invention uses a polypeptide or a polypeptide encoded by a polynucleotide in animal feed having at least 87% sequence identity to the polypeptide of SEQ ID NO: 2.

An embodiment of the invention uses a polypeptide or a polypeptide encoded by a polynucleotide in animal feed having at least 88% sequence identity to the polypeptide of SEQ ID NO: 2.

An embodiment of the invention uses a polypeptide or a polypeptide encoded by a polynucleotide in animal feed having at least 89% sequence identity to the polypeptide of SEQ ID NO: 2.

An embodiment of the invention uses a polypeptide or a polypeptide encoded by a polynucleotide in animal feed having at least 90% sequence identity to the polypeptide of SEQ ID NO: 2.

An embodiment of the invention uses a polypeptide or a polypeptide encoded by a polynucleotide for use in animal feed or detergents having at least 91% sequence identity to the polypeptide of SEQ ID NO: 2.

An embodiment of the invention uses a polypeptide or a polypeptide encoded by a polynucleotide in animal feed having at least 92% sequence identity to the polypeptide of SEQ ID NO: 2.

An embodiment of the invention uses a polypeptide or a polypeptide encoded by a polynucleotide in animal feed having at least 93% sequence identity to the polypeptide of SEQ ID NO: 2.

An embodiment of the invention uses a polypeptide or a polypeptide encoded by a polynucleotide in animal feed having at least 94% sequence identity to the polypeptide of SEQ ID NO: 2.

An embodiment of the invention uses a polypeptide or a polypeptide encoded by a polynucleotide in animal feed having at least 95% sequence identity to the polypeptide of SEQ ID NO: 2.

An embodiment of the invention uses a polypeptide or a polypeptide encoded by a polynucleotide in animal feed having at least 96% sequence identity to the polypeptide of SEQ ID NO: 2.

An embodiment of the invention uses a polypeptide or a polypeptide encoded by a polynucleotide in animal feed having at least 97% sequence identity to the polypeptide of SEQ ID NO: 2.

An embodiment of the invention uses a polypeptide or a polypeptide encoded by a polynucleotide in animal feed having at least 98% sequence identity to the polypeptide of SEQ ID NO: 2.

An embodiment of the invention uses a polypeptide or a polypeptide encoded by a polynucleotide in animal feed having at least 99% sequence identity to the polypeptide of SEQ ID NO: 2.

An embodiment of the invention uses a polypeptide or a polypeptide encoded by a polynucleotide in animal feed having 100% sequence identity to the polypeptide of SEQ ID NO: 2.

Each of the above embodiments also relate to not only SEQ ID NO:2, but also to any of SEQ ID NO:4, 5 and 6.

Consequently the invention also relates to:

An embodiment of the invention uses a polypeptide or a polypeptide encoded by a polynucleotide in animal feed having at least 80% sequence identity to the polypeptide of SEQ ID NO: 4.

An embodiment of the invention uses a polypeptide or a polypeptide encoded by a polynucleotide in animal feed having at least 82% sequence identity to the polypeptide of SEQ ID NO: 4.

An embodiment of the invention uses a polypeptide or a polypeptide encoded by a polynucleotide in animal feed having at least 84% sequence identity to the polypeptide of SEQ ID NO: 4.

An embodiment of the invention uses a polypeptide or a polypeptide encoded by a polynucleotide in animal feed having at least 85% sequence identity to the polypeptide of SEQ ID NO: 4.

An embodiment of the invention uses a polypeptide or a polypeptide encoded by a polynucleotide in animal feed having at least 86% sequence identity to the polypeptide of SEQ ID NO: 4.

An embodiment of the invention uses a polypeptide or a polypeptide encoded by a polynucleotide in animal feed having at least 87% sequence identity to the polypeptide of SEQ ID NO: 4.

An embodiment of the invention uses a polypeptide or a polypeptide encoded by a polynucleotide in animal feed having at least 88% sequence identity to the polypeptide of SEQ ID NO: 4.

An embodiment of the invention uses a polypeptide or a polypeptide encoded by a polynucleotide in animal feed having at least 89% sequence identity to the polypeptide of SEQ ID NO: 4.

An embodiment of the invention uses a polypeptide or a polypeptide encoded by a polynucleotide in animal feed having at least 90% sequence identity to the polypeptide of SEQ ID NO: 4.

An embodiment of the invention uses a polypeptide or a polypeptide encoded by a polynucleotide for use in animal feed or detergents having at least 91% sequence identity to the polypeptide of SEQ ID NO: 4.

An embodiment of the invention uses a polypeptide or a polypeptide encoded by a polynucleotide in animal feed having at least 92% sequence identity to the polypeptide of SEQ ID NO: 4.

An embodiment of the invention uses a polypeptide or a polypeptide encoded by a polynucleotide in animal feed having at least 93% sequence identity to the polypeptide of SEQ ID NO: 4.

An embodiment of the invention uses a polypeptide or a polypeptide encoded by a polynucleotide in animal feed having at least 94% sequence identity to the polypeptide of SEQ ID NO: 4.

An embodiment of the invention uses a polypeptide or a polypeptide encoded by a polynucleotide in animal feed having at least 95% sequence identity to the polypeptide of SEQ ID NO: 4.

An embodiment of the invention uses a polypeptide or a polypeptide encoded by a polynucleotide in animal feed having at least 96% sequence identity to the polypeptide of SEQ ID NO: 4.

An embodiment of the invention uses a polypeptide or a polypeptide encoded by a polynucleotide in animal feed having at least 97% sequence identity to the polypeptide of SEQ ID NO: 4.

An embodiment of the invention uses a polypeptide or a polypeptide encoded by a polynucleotide in animal feed having at least 98% sequence identity to the polypeptide of SEQ ID NO: 4.

An embodiment of the invention uses a polypeptide or a polypeptide encoded by a polynucleotide in animal feed having at least 99% sequence identity to the polypeptide of SEQ ID NO: 4.

An embodiment of the invention uses a polypeptide or a polypeptide encoded by a polynucleotide in animal feed having 100% sequence identity to the polypeptide of SEQ ID NO: 4.

And

An embodiment of the invention uses a polypeptide or a polypeptide encoded by a polynucleotide in animal feed having at least 80% sequence identity to the polypeptide of SEQ ID NO: 5.

An embodiment of the invention uses a polypeptide or a polypeptide encoded by a polynucleotide in animal feed having at least 82% sequence identity to the polypeptide of SEQ ID NO: 5.

An embodiment of the invention uses a polypeptide or a polypeptide encoded by a polynucleotide in animal feed having at least 84% sequence identity to the polypeptide of SEQ ID NO: 5.

An embodiment of the invention uses a polypeptide or a polypeptide encoded by a polynucleotide in animal feed having at least 85% sequence identity to the polypeptide of SEQ ID NO: 5.

An embodiment of the invention uses a polypeptide or a polypeptide encoded by a polynucleotide in animal feed having at least 86% sequence identity to the polypeptide of SEQ ID NO: 5.

An embodiment of the invention uses a polypeptide or a polypeptide encoded by a polynucleotide in animal feed having at least 87% sequence identity to the polypeptide of SEQ ID NO: 5.

An embodiment of the invention uses a polypeptide or a polypeptide encoded by a polynucleotide in animal feed having at least 88% sequence identity to the polypeptide of SEQ ID NO: 5.

An embodiment of the invention uses a polypeptide or a polypeptide encoded by a polynucleotide in animal feed having at least 89% sequence identity to the polypeptide of SEQ ID NO: 5.

An embodiment of the invention uses a polypeptide or a polypeptide encoded by a polynucleotide in animal feed having at least 90% sequence identity to the polypeptide of SEQ ID NO: 5.

An embodiment of the invention uses a polypeptide or a polypeptide encoded by a polynucleotide for use in animal feed or detergents having at least 91% sequence identity to the polypeptide of SEQ ID NO: 5.

An embodiment of the invention uses a polypeptide or a polypeptide encoded by a polynucleotide in animal feed having at least 92% sequence identity to the polypeptide of SEQ ID NO: 5.

An embodiment of the invention uses a polypeptide or a polypeptide encoded by a polynucleotide in animal feed having at least 93% sequence identity to the polypeptide of SEQ ID NO: 5.

An embodiment of the invention uses a polypeptide or a polypeptide encoded by a polynucleotide in animal feed having at least 94% sequence identity to the polypeptide of SEQ ID NO: 5.

An embodiment of the invention uses a polypeptide or a polypeptide encoded by a polynucleotide in animal feed having at least 95% sequence identity to the polypeptide of SEQ ID NO: 5.

An embodiment of the invention uses a polypeptide or a polypeptide encoded by a polynucleotide in animal feed having at least 96% sequence identity to the polypeptide of SEQ ID NO: 5.

An embodiment of the invention uses a polypeptide or a polypeptide encoded by a polynucleotide in animal feed having at least 97% sequence identity to the polypeptide of SEQ ID NO: 5.

An embodiment of the invention uses a polypeptide or a polypeptide encoded by a polynucleotide in animal feed having at least 98% sequence identity to the polypeptide of SEQ ID NO: 5.

An embodiment of the invention uses a polypeptide or a polypeptide encoded by a polynucleotide in animal feed having at least 99% sequence identity to the polypeptide of SEQ ID NO: 5.

An embodiment of the invention uses a polypeptide or a polypeptide encoded by a polynucleotide in animal feed having 100% sequence identity to the polypeptide of SEQ ID NO: 2.

And

An embodiment of the invention uses a polypeptide or a polypeptide encoded by a polynucleotide in animal feed having at least 80% sequence identity to the polypeptide of SEQ ID NO: 6.

An embodiment of the invention uses a polypeptide or a polypeptide encoded by a polynucleotide in animal feed having at least 82% sequence identity to the polypeptide of SEQ ID NO: 6.

An embodiment of the invention uses a polypeptide or a polypeptide encoded by a polynucleotide in animal feed having at least 84% sequence identity to the polypeptide of SEQ ID NO: 6.

An embodiment of the invention uses a polypeptide or a polypeptide encoded by a polynucleotide in animal feed having at least 85% sequence identity to the polypeptide of SEQ ID NO: 6.

An embodiment of the invention uses a polypeptide or a polypeptide encoded by a polynucleotide in animal feed having at least 86% sequence identity to the polypeptide of SEQ ID NO: 6.

An embodiment of the invention uses a polypeptide or a polypeptide encoded by a polynucleotide in animal feed having at least 87% sequence identity to the polypeptide of SEQ ID NO: 6.

An embodiment of the invention uses a polypeptide or a polypeptide encoded by a polynucleotide in animal feed having at least 88% sequence identity to the polypeptide of SEQ ID NO: 6.

An embodiment of the invention uses a polypeptide or a polypeptide encoded by a polynucleotide in animal feed having at least 89% sequence identity to the polypeptide of SEQ ID NO: 6.

An embodiment of the invention uses a polypeptide or a polypeptide encoded by a polynucleotide in animal feed having at least 90% sequence identity to the polypeptide of SEQ ID NO: 6.

An embodiment of the invention uses a polypeptide or a polypeptide encoded by a polynucleotide for use in animal feed or detergents having at least 91% sequence identity to the polypeptide of SEQ ID NO: 6.

An embodiment of the invention uses a polypeptide or a polypeptide encoded by a polynucleotide in animal feed having at least 92% sequence identity to the polypeptide of SEQ ID NO: 6.

An embodiment of the invention uses a polypeptide or a polypeptide encoded by a polynucleotide in animal feed having at least 93% sequence identity to the polypeptide of SEQ ID NO: 6.

An embodiment of the invention uses a polypeptide or a polypeptide encoded by a polynucleotide in animal feed having at least 94% sequence identity to the polypeptide of SEQ ID NO: 6.

An embodiment of the invention uses a polypeptide or a polypeptide encoded by a polynucleotide in animal feed having at least 95% sequence identity to the polypeptide of SEQ ID NO: 6.

An embodiment of the invention uses a polypeptide or a polypeptide encoded by a polynucleotide in animal feed having at least 96% sequence identity to the polypeptide of SEQ ID NO: 6.

An embodiment of the invention uses a polypeptide or a polypeptide encoded by a polynucleotide in animal feed having at least 97% sequence identity to the polypeptide of SEQ ID NO: 6.

An embodiment of the invention uses a polypeptide or a polypeptide encoded by a polynucleotide in animal feed having at least 98% sequence identity to the polypeptide of SEQ ID NO: 6.

An embodiment of the invention uses a polypeptide or a polypeptide encoded by a polynucleotide in animal feed having at least 99% sequence identity to the polypeptide of SEQ ID NO: 6.

An embodiment of the invention uses a polypeptide or a polypeptide encoded by a polynucleotide in animal feed having 100% sequence identity to the polypeptide of SEQ ID NO: 6.

A polypeptide to be used in the present invention preferably comprises or consists of the amino acid sequence of SEQ ID NO: 2, SEQ ID NO: 4, SEQ ID NO:5 and/or SEQ ID NO: 6 or an allelic variant thereof; or is a fragment thereof missing e.g. 30, 25, 20, 15, 10 or 5 amino acids from the *N*- and/or C-terminal and having $\alpha$-L- galactosidase activity. In another aspect, the polypeptide comprises or consists of the mature polypeptide of SEQ ID NO: 2, SEQ ID NO: 4 SEQ ID NO:5 and/or SEQ ID NO: 6 . In another aspect, the polypeptide comprises or consists of amino acids 22 to 814 of SEQ ID NO: 2, amino acids 22 to 814 of SEQ ID NO: 4, SEQ ID NO:5 and/or amino acids 22 to 814 of SEQ ID NO: 6.

In another embodiment, the present invention relates to an isolated polypeptide having $\alpha$-L-galactosidase activity encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, or (ii) the full-length complement of (i) (Sambrook et al., 1989, Molecular Cloning, A Laboratory Manual, 2d edition, Cold Spring Harbor, New York).

The polynucleotide of SEQ ID NO: 1 or a subsequence thereof, as well as the polypeptide of SEQ ID NO: 2 or a fragment thereof, may be used to design nucleic acid probes to identify and clone DNA encoding polypeptides having $\alpha$-L-galactosidase activity from strains of different genera or species according to methods well known in the art. In particular, such probes can be used for hybridization with the genomic DNA or cDNA of a cell of interest, following standard Southern blotting procedures, in order to identify and isolate the corresponding gene therein. Such probes can be considerably shorter than the entire sequence, but should be at least 15, e.g., at least 25, at least 35, or at least 70 nucleotides in length. Preferably, the nucleic acid probe is at least 100 nucleotides in length, e.g., at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}$P, $^{3}$H, $^{35}$S, biotin, or avidin). Such probes are encompassed by the present invention.

A genomic DNA or cDNA library prepared from such other strains may be screened for DNA that hybridizes with the probes described above and encodes a polypeptide having $\alpha$-L-galactosidase activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that hybridizes with SEQ ID NO: 1 or a subsequence thereof, the carrier material is used in a Southern blot.

For purposes of the present invention, hybridization indicates that the polynucleotide hybridizes to a labeled nucleic acid probe corresponding to (i) SEQ ID NO: 1; (ii) the mature polypeptide coding sequence of SEQ ID NO: 1; (iii) the full-length complement thereof; or (iv) a subsequence thereof; under very low to very high stringency conditions. Molecules to which the nucleic acid probe hybridizes under these conditions can be detected using, for example, X-ray film or any other detection means known in the art.

In one aspect, the nucleic acid probe is a polynucleotide that encodes the polypeptide of SEQ ID NO: 2; the mature polypeptide thereof; or a fragment thereof. In another aspect, the nucleic acid probe is SEQ ID NO: 1. In another aspect, the nucleic acid probe is the mature polypeptide coding region contained in B. *Ovatum* ATCC - 8483, wherein the polynucleotide encodes a polypeptide having $\alpha$-L-galactosidase activity.

For long probes of at least 100 nucleotides in length, high to very high stringency conditions are defined as prehybridization and hybridization at 42°C in 5X SSPE, 0.3% SDS, 200 micrograms/ml sheared and denatured salmon sperm DNA, and either 25% formamide for very low and low stringencies, 35% formamide for medium and medium-high stringencies, or 50% formamide for high and very high stringencies, following standard Southern blotting procedures for 12 to 24 hours optimally. The carrier material is finally washed three times each for 15 minutes using 2X SSC, 0.2% SDS at 65°C (high stringency), and at 70°C (very high stringency).

For short probes of about 15 nucleotides to about 70 nucleotides in length, stringency conditions are defined as prehybridization and hybridization at about 5°C to about 10°C below the calculated $T_m$ using the calculation according to Bolton and McCarthy (1962, Proc. Natl. Acad. Sci. USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures for 12 to 24 hours optimally. The carrier material is finally washed once in 6X SCC plus 0.1 % SDS for 15 minutes and twice each for 15 minutes using 6X SSC at 5°C to 10°C below the calculated $T_m$.

In another embodiment, the present invention relates to an isolated polypeptide having $\alpha$-L-galactosidase activity encoded by a polynucleotide having a sequence identity to the mature polypeptide coding sequence of SEQ ID NO: 1 at least 92%, at least 93%, at least 94%, at least 95%, at least 96%, at least 97%, at least 98%, at least 99%, or 100%.

In another embodiment, the present invention relates to variants of the mature polypeptide of SEQ ID NO: 2 comprising a substitution, deletion, and/or insertion at one or more (e.g., several) positions. In an embodiment, the number of amino acid substitutions, deletions and/or insertions introduced into the mature polypeptide of SEQ ID NO: 2 is up to 10, e.g., 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for $\alpha$-L-galactosidase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

GH95 enzymes are considered to employ a unique reaction mechanism, in which Asp-activated Asn acts as a general base catalyst involving Glu566 and Asn423 (Nagae M, Tsuchiya A, Katayama T, Yamamoto K, Wakatsuki S, and Kato R. Structural basis of the catalytic reaction mechanism of novel 1,2-alpha-L-fucosidase from Bifidobacterium bifidum. J Biol Chem. 2007 Jun 22;282(25):18497-509.. Glu566 is hydrogen-bonded with Asn421. Asn423 is activated by the neighboring Asp766, and consequently activates a nucleophilic water molecule. This model (carboxylic acid-mediated activation of amido group) bears some analogy to the neighboring group participation mechanism employed by members of GH18, GH20, GH25, GH56, GH84 and GH85. These four residues are invariable in the members of this family, and substitution with alanine or glycine diminishes activities by 1,000- to 10,000-fold (Nagae M, Tsuchiya A, Katayama T, Yamamoto K, Wakatsuki S, and Kato R. Structural basis of the catalytic reaction mechanism of novel 1,2-alpha-L-fucosidase from Bifidobacterium bifidum. J Biol Chem. 2007 Jun 22;282(25):18497-509.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

## Sources of Polypeptides Having α-L-Galactosidase Activity

A polypeptide having α-L-galactosidase activity of the present invention may be obtained from microorganisms of any genus. For purposes of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

The polypeptide may be a bacterial polypeptide. For example, the polypeptide may be a Gram-negative bacterial polypeptide such as a *Bacteroides, Campylobacter, E. coli, Flavobacterium, Fusobacterium, Helicobacter, Ilyobacter, Neisseria, Pseudomonas, Salmonella,* or *Ureaplasma* polypeptide or a Gram-positive bacterial polypeptide such as a *Bacillus, Clostridium, Enterococcus, Geobacillus, Lactobacillus, Lactococcus, Oceanobacillus, Staphylococcus, Streptococcus,* or *Streptomyces* polypeptide having alpha xylosidase activity.

In one aspect, the polypeptide is a *Bacteroides ovatus, Bacteroides acidifaciens, Bacteroides gracilis, Bacteroides oris, Bacteroides putredinis, Bacteroides pyogenes* or *Bacteroides vulgatus* polypeptide.

In another aspect, the polypeptide is a *Bacteroides ovatus* polypeptide, *e.g.,* a polypeptide obtained from *Bacteroides ovatus ATCC 8483.*

In one aspect, the polypeptide is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis* polypeptide.

In another aspect, the polypeptide is a *Streptococcus equisimilis, Streptococcus pyogenes, Streptococcus uberis,* or *Streptococcus equi* subsp. *Zooepidemicus* polypeptide.

In another aspect, the polypeptide is a *Streptomyces achromogenes, Streptomyces avermitilis, Streptomyces coelicolor, Streptomyces griseus,* or *Streptomyces lividans* polypeptide.

The polypeptide may be a fungal polypeptide. For example, the polypeptide may be a yeast polypeptide such as a *Candida, Kluyveromyces, Pichia, Saccharomyces, Schizosaccharomyces,* or *Yarrowia* polypeptide; or a filamentous fungal polypeptide such as an *Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* polypeptide.

In another aspect, the polypeptide is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* polypeptide.

In another aspect, the polypeptide is an *Acremonium cellulolyticus, Aspergillus aculeatus, Aspergillus awamori, Aspergillus foetidus, Aspergillus fumigatus, Aspergillus japonicus, Aspergillus nidulans, Aspergillus niger, Aspergillus oryzae, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia setosa, Thielavia spededonium, Thielavia subthermophila, Thielavia terrestris, Trichoderma harzianum, Trichoderma koningii, Trichoderma longibrachiatum, Trichoderma reesei,* or *Trichoderma viride* polypeptide.

It will be understood that for the aforementioned species, the invention encompasses both the perfect and imperfect states, and other taxonomic equivalents, *e.g.,* anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The polypeptide may be identified and obtained from other sources including microorganisms isolated from nature *(e.g.,* soil, composts, water, etc.) or DNA samples obtained directly from natural materials *(e.g.,* soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art *(see, e.g.,* Sambrook *et al.,* 1989, *supra).*

**Catalytic Domains**

In one embodiment, the present invention also relates to catalytic domains having a sequence identity to amino acids 20 to 811 of SEQ ID NO: 2 of at least least 80%, at least 85%, at least 86%, at least 87%, at least 88%, at least 89%, least 90%, at least 91%, at least 92%, at least 93%, at least 94%, at least 95%, at least 96%, at least 97%, at least 98%, at least 99%, least 92%, at least 95%, at least 96%, at least 97%, at least 98%, at least 99%, or 100%. In one aspect, the catalytic domains comprise amino acid sequences that differ by up to 10 amino acids, *e.g.,* 1, 2, 3, 4, 5, 6, 7, 8, 9, or 10, from amino acids 20 to 811 of SEQ ID NO: 2.

The catalytic domain preferably comprises or consists of amino acids 20 to 811 of SEQ ID NO: 2 or an allelic variant thereof; or is a fragment thereof having α-L-galactosidase activity.

In another embodiment, the present invention also relates to catalytic domains encoded by polynucleotides that hybridize under very low stringency conditions, low stringency conditions, medium stringency conditions, medium-high stringency conditions, high stringency conditions, or very high stringency conditions (as defined above) with (i) the nucleotides 60 to of SEQ ID NO: 1, or (ii) the full-length complement of (i) (Sambrook *et al.,* 1989, *supra*).

In another embodiment, the present invention also relates to catalytic domains encoded by polynucleotides having a sequence identity to nucleotides 60 to 2433 of SEQ ID NO: 1 at least 80%, at least 85%, at least 86%, at least 87%, at least 88%, at least 89%, least 90%, at least 91%, at least 92%, at least 93%, at least 94%, at least 95%, at least 96%, at least 97%, at least 98%, at least 99%, least 92%, at least 95%, at least 96%, at least 97%, at least 98%, at least 99%, or 100%.

The polynucleotide encoding the catalytic domain preferably comprises or consists of nucleotides 60 to 2433 of SEQ ID NO: 1 or is the sequence contained in ATCC 8483.

In another embodiment, the present invention also relates to catalytic domain variants of amino acids 20 to 811 of SEQ ID NO: 2 comprising a substitution, deletion, and/or insertion at one or more (e.g., several) positions. In one aspect, the number of amino acid substitutions, deletions and/or insertions introduced into the sequence of amino acids 20 to 811 of SEQ ID NO: 2 is up to 10, e.g., 1,2,3,4, 5, 6, 8, 9, or 10.

**Polynucleotides**

The present invention also relates to isolated polynucleotides encoding a polypeptide, a catalytic domain of the present invention, as described herein.

The techniques used to isolate or clone a polynucleotide are known in the art and include isolation from genomic DNA or cDNA, or a combination thereof. The cloning of the polynucleotides from genomic DNA can be effected, *e.g.,* by using the well known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of *Bacteroides,* or a related organism and thus, for example, may be an allelic or species variant of the polypeptide encoding region of the polynucleotide.

Modification of a polynucleotide encoding a polypeptide of the present invention may be necessary for synthesizing polypeptides substantially similar to the polypeptide. The term "substantially similar" to the polypeptide refers to non-naturally occurring forms of the polypeptide. These polypeptides may differ in some engineered way from the polypeptide isolated from its native source, *e.g.,* variants that differ in specific activity, thermostability, pH optimum, or the like. The variants may be constructed on the basis of the polynucleotide presented as the mature polypeptide coding sequence of SEQ ID NO: 1, *e.g.,* a subsequence thereof, and/or by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, *see, e.g.,* Ford etal., 1991, Protein Expression and Purification 2: 95-107.

**Nucleic Acid Constructs**

Also disclosed are nucleic acid constructs comprising a polynucleotide of the present invention operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs here disclosed in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene *(dagA),* and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs here disclosed in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase *(glaA), Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase *I, Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease *(aprH), Bacillus licheniformis* alpha-amylase *(amyL),* and *Escherichia coli* ribosomal RNA *(rrnB).*

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM),* and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus* nigerglucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

**Expression Vectors**

Also disclosed are recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.,* a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosylaminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is an *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra*).

**Host Cells**

Here disclosed are recombinant host cells, comprising a polynucleotide of the present invention operably linked to one or more control sequences that direct the production of a polypeptide of the present invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, e.g., a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi* subsp. *Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, e.g., Chang and Cohen, 1979, Mol. Gen. Genet. 168: 111-115), competent cell transformation (see, *e.g.*, Young and Spizizen, 1961, J. Bacteriol. 81: 823-829, or Dubnau and Davidoff-Abelson, 1971, J. Mol. Biol. 56: 209-221), electroporation (see, *e.g.*, Shigekawa and Dower, 1988, Biotechniques 6: 742-751), or conjugation (see, *e.g.*, Koehler and Thorne, 1987, J. Bacteriol. 169: 5271-5278). The introduction of DNA into an *E. coli* cell may be effected by protoplast transformation (see, *e.g.*, Hanahan, 1983, J. Mol. Biol. 166: 557-580) or electroporation (see, *e.g.*, Dower et al., 1988, Nucleic Acids Res. 16: 6127-6145). The introduction of DNA into a *Streptomyces* cell may be effected by protoplast transformation, electroporation (see, *e.g.*, Gong et al., 2004, Folia Microbiol. (Praha) 49: 399-405), conjugation (see, *e.g.*, Mazodier et al., 1989, J. Bacteriol. 171: 3583-3585), or transduction (see, *e.g.*, Burke et al., 2001, Proc. Natl. Acad. Sci. USA 98: 6289-6294). The introduction of DNA into a *Pseudomonas* cell may be effected by electroporation (see, *e.g.*, Choi et al., 2006, J. Microbiol. Methods 64: 391-397) or conjugation (see, *e.g.*, Pinedo and Smets, 2005, Appl. Environ. Microbiol. 71: 51-57). The introduction of DNA into a *Streptococcus* cell may be effected by natural competence (see, *e.g.*, Perry and Kuramitsu, 1981, Infect. Immun. 32: 1295-1297), protoplast transformation (see, *e.g.*, Catt and Jollick, 1991, Microbios 68: 189-207), electroporation (see, *e.g.*, Buckley et al., 1999, Appl. Environ. Microbiol. 65: 3800-3804), or conjugation (see, *e.g.*, Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceripori-*

*opsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

## Methods of Production

Also disclosed are methods of producing a polypeptide of the present invention, comprising (a) cultivating a cell, which in its wild-type form produces the polypeptide, under conditions conducive for production of the polypeptide; and optionally, (b) recovering the polypeptide. In one aspect, the cell is a *Bacteroides* cell. In another aspect, the cell is a *Bacteroides ovatus* cell. In another aspect, the cell is *Bacteroides ovatus* ATCC8483. Disclosed are methods of producing a polypeptide of the present invention, comprising (a) cultivating a recombinant host cell of the present invention under conditions conducive for production of the polypeptide; and optionally, (b) recovering the polypeptide.

The host cells are cultivated in a nutrient medium suitable for production of the polypeptide using methods known in the art. For example, the cells may be cultivated by shake flask cultivation, or small-scale or large-scale fermentation (including continuous, batch, fed-batch, or solid state fermentations) in laboratory or industrial fermentors in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods include, but are not limited to, use of specific antibodies, formation of an enzyme product, or disappearance of an enzyme substrate. For example, an enzyme assay measuring the release of L-galactose from corn (maize) bran xylan may be used to determine the activity of the polypeptide or to measure the enzymatic release of para nitro phenol from an artificial PNP $\alpha$-L-galactose substrate.

The polypeptide may be recovered using methods known in the art. For example, the polypeptide may be recovered from the nutrient medium by conventional procedures including, but not limited to, collection, centrifugation, filtration, extraction, spray-drying, evaporation, or precipitation. In one aspect, a fermentation broth comprising the polypeptide is recovered.

The polypeptide may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.*, Protein Purification, Janson and Ryden, editors, VCH Publishers, New York, 1989) to obtain substantially pure polypeptides.

In an alternative aspect, the polypeptide is not recovered, but rather a host cell of the present invention expressing the polypeptide is used as a source of the polypeptide.

**Removal or Reduction of α L-Galactosidase Activity**

Here disclosed are also methods of producing a mutant of a parent cell, which comprises disrupting or deleting a polynucleotide, or a portion thereof, encoding a polypeptide of the present invention, which results in the mutant cell producing less of the polypeptide than the parent cell when cultivated under the same conditions.

The mutant cell may be constructed by reducing or eliminating expression of the polynucleotide using methods well known in the art, for example, insertions, disruptions, replacements, or deletions. In a preferred aspect, the polynucleotide is inactivated. The polynucleotide to be modified or inactivated may be, for example, the coding region or a part thereof essential for activity, or a regulatory element required for expression of the coding region. An example of such a regulatory or control sequence may be a promoter sequence or a functional part thereof, *i.e.,* a part that is sufficient for affecting expression of the polynucleotide. Other control sequences for possible modification include, but are not limited to, a leader, polyadenylation sequence, propeptide sequence, signal peptide sequence, transcription terminator, and transcriptional activator.

Modification or inactivation of the polynucleotide may be performed by subjecting the parent cell to mutagenesis and selecting for mutant cells in which expression of the polynucleotide has been reduced or eliminated. The mutagenesis, which may be specific or random, may be performed, for example, by use of a suitable physical or chemical mutagenizing agent, by use of a suitable oligonucleotide, or by subjecting the DNA sequence to PCR generated mutagenesis. Furthermore, the mutagenesis may be performed by use of any combination of these mutagenizing agents.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues.

When such agents are used, the mutagenesis is typically performed by incubating the parent cell to be mutagenized in the presence of the mutagenizing agent of choice under suitable conditions, and screening and/or selecting for mutant cells exhibiting reduced or no expression of the gene.

Modification or inactivation of the polynucleotide may be accomplished by insertion, substitution, or deletion of one or more nucleotides in the gene or a regulatory element required for transcription or translation thereof. For example, nucleotides may be inserted or removed so as to result in the introduction of a stop codon, the removal of the start codon, or a change in the open reading frame. Such modification or inactivation may be accomplished by site-directed mutagenesis or PCR generated mutagenesis in accordance with methods known in the art. Although, in principle, the modification may be performed *in vivo, i.e.,* directly on the cell expressing the polynucleotide to be modified, it is preferred that the modification be performed *in vitro* as exemplified below.

An example of a convenient way to eliminate or reduce expression of a polynucleotide is based on techniques of gene replacement, gene deletion, or gene disruption. For example, in the gene disruption method, a nucleic acid sequence corresponding to the endogenous polynucleotide is mutagenized *in vitro* to produce a defective nucleic acid sequence that is then transformed into the parent cell to produce a defective gene. By homologous recombination, the defective nucleic acid sequence replaces the endogenous polynucleotide. It may be desirable that the defective polynucleotide also encodes a marker that may be used for selection of transformants in which the polynucleotide has been modified or destroyed. In an aspect, the polynucleotide is disrupted with a selectable marker such as those described herein. Also disclosed are methods of inhibiting the expression of a polypeptide having α-L-galactosidase activity in a cell, comprising administering to the cell or expressing in the cell a double-stranded RNA (dsRNA) molecule, wherein the dsRNA comprises a subsequence of a polynucleotide of the present invention. In a preferred aspect, the dsRNA is about 15, 16, 17, 18, 19, 20, 21, 22, 23, 24, 25 or more duplex nucleotides in length.

The dsRNA is preferably a small interfering RNA (siRNA) or a micro RNA (miRNA). In a preferred aspect, the dsRNA is small interfering RNA for inhibiting transcription. In another preferred aspect, the dsRNA is micro RNA for inhibiting translation. Also disclosed are double-stranded RNA (dsRNA) molecules, comprising a portion of the mature polypeptide coding sequence of SEQ ID NO: 1 for inhibiting expression of the polypeptide in a cell. While the present invention is not limited by any particular mechanism of action, the dsRNA can enter a cell and cause the degradation of a single-stranded RNA (ssRNA) of similar or identical sequences, including endogenous mRNAs. When a cell is exposed to dsRNA, mRNA from the homologous gene is selectively degraded by a process called RNA interference (RNAi).

The dsRNAs can be used in gene-silencing. In one aspect, provided are methods to selectively degrade RNA using a dsRNAi here disclosed. The process may be practiced *in vitro, ex vivo* or *in vivo.* In one aspect, the dsRNA molecules can be used to generate a loss-of-function mutation in a cell, an organ or an animal. Methods for making and using dsRNA molecules to selectively degrade RNA are well known in the art; see, for example, U.S. Patent Nos. 6,489,127; 6,506,559; 6,511,824; and 6,515,109. Also disclosed is a mutant cell of a parent cell that comprises a disruption or deletion of a polynucleotide encoding the polypeptide or a control sequence thereof or a silenced gene encoding the polypeptide, which results in the mutant cell producing less of the polypeptide or no polypeptide compared to the parent cell.

The polypeptide-deficient mutant cells are particularly useful as host cells for expression of native and heterologous polypeptides. Therefore, the present invention further relates to methods of producing a native or heterologous polypeptide, comprising (a) cultivating the mutant cell under conditions conducive for production of the polypeptide; and (b) recovering the polypeptide. The term "heterologous polypeptides" means polypeptides that are not native to the host cell, *e.g.*, a variant of a native protein. The host cell may comprise more than one copy of a polynucleotide encoding the native or heterologous polypeptide.

The methods used for cultivation and purification of the product of interest may be performed by methods known in the art.

The methods here disclosed for producing an essentially α-L-galactosidase-free product are of particular interest in the production of eukaryotic polypeptides, in particular fungal proteins such as enzymes. The α-L-galactosidase-deficient cells may also be used to express heterologous proteins of pharmaceutical interest such as hormones, growth factors, receptors, and the like. The term "eukaryotic polypeptides" includes not only native polypeptides, but also those polypeptides, *e.g.*, enzymes, which have been modified by amino acid substitutions, deletions or additions, or other such modifications to enhance activity, thermostability, pH tolerance and the like.

In a further aspect, also disclosed is a protein product essentially free from α-L-galactosidase activity that is produced by a method of the present invention.

**Fermentation Broth Formulations or Cell Compositions**

The present invention also relates to a fermentation broth formulation or a cell composition comprising a polypeptide of the present invention. The fermentation broth product further comprises additional ingredients used in the fermentation process, such as, for example, cells (including, the host cells containing the gene encoding the polypeptide of the present invention which are used to produce the polypeptide of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.*, expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.*, filamentous fungal cells) are removed, *e.g.*, by centrifugation. In some embodiments, the fermentation broth contains spent cell culture medium, extracellular enzymes, and viable and/or nonviable microbial cells.

In an embodiment, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific embodiment, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In one embodiment, the killed cells and/or cell debris are removed from a cell-killed whole broth to provide a composition that is free of these components.

The fermentation broth formulations or cell compositions may further comprise a preservative and/or anti-microbial (*e.g.*, bacteriostatic) agent, including, but not limited to, sorbitol, sodium chloride, potassium sorbate, and others known in the art.

The cell-killed whole broth or composition may contain the unfractionated contents of the fermentation materials derived at the end of the fermentation. Typically, the cell-killed whole broth or composition contains the spent culture medium and cell debris present after the microbial cells (*e.g.*, filamentous fungal cells) are grown to saturation, incubated under carbon-limiting conditions to allow protein synthesis. In some embodiments, the cell-killed whole broth or composition contains the spent cell culture medium, extracellular enzymes, and killed filamentous fungal cells. In some embodiments, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673.

**Enzyme Compositions**

The present invention also relates to compositions comprising a polypeptide of the present invention. Preferably, the compositions are enriched in such a polypeptide. The term "enriched" indicates that the α-L-galactosidase activity of the composition has been increased, e.g., with an enrichment factor of at least 1.1.

In one aspect, the composition comprises an isolated polypeptide having α-L-galactosidase activity, selected from the group consisting of:

(a) a polypeptide having at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 3 (No. 1-792);

(b) a polypeptide encoded by a polynucleotide that hybridizes under high stringency conditions, or very high stringency conditions with:

(i) the mature polypeptide coding sequence of SEQ ID NO: 1; and/or
(iii) the full-length complementary strand of (i);

(c) a polypeptide encoded by a polynucleotide having at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 1;

(d) a variant comprising a substitution, deletion, and/or insertion of one or more (several) amino acids of the mature polypeptide of SEQ ID NO: 3; and

(e) a fragment of a polypeptide of (a), (b), (c) or (d), that has α-xylosidase activity.

An embodiment of the invention is a composition comprising an isolated polypeptide having at least 85% sequence identity to the mature polypeptide of SEQ ID NO: 3.

An embodiment of the invention is a composition comprising an isolated polypeptide having at least 86% sequence identity to the mature polypeptide of SEQ ID NO: 3.

An embodiment of the invention is a composition comprising an isolated polypeptide having at least 87% sequence identity to the mature polypeptide of SEQ ID NO: 3.

An embodiment of the invention is a composition comprising an isolated polypeptide having at least 88% sequence identity to the mature polypeptide of SEQ ID NO: 3.

An embodiment of the invention is a composition comprising an isolated polypeptide having at least 89% sequence identity to the mature polypeptide of SEQ ID NO: 3.

An embodiment of the invention is a composition comprising an isolated polypeptide having at least 90% sequence identity to the mature polypeptide of SEQ ID NO: 3.

An embodiment of the invention is a composition comprising an isolated polypeptide having at least 91% sequence identity to the mature polypeptide of SEQ ID NO: 3.

An embodiment of the invention is a composition comprising an isolated polypeptide having at least 92% sequence identity to the mature polypeptide of SEQ ID NO: 3.

An embodiment of the invention is a composition comprising an isolated polypeptide having at least 93% sequence identity to the mature polypeptide of SEQ ID NO: 3.

An embodiment of the invention is a composition comprising an isolated polypeptide having at least 94% sequence identity to the mature polypeptide of SEQ ID NO: 3.

An embodiment of the invention is a composition comprising an isolated polypeptide having at least 95% sequence identity to the mature polypeptide of SEQ ID NO: 3.

An embodiment of the invention is a composition comprising an isolated polypeptide having at least 96% sequence identity to the mature polypeptide of SEQ ID NO: 3.

An embodiment of the invention is a composition comprising an isolated polypeptide having at least 97% sequence identity to the mature polypeptide of SEQ ID NO: 3.

An embodiment of the invention is a composition comprising an isolated polypeptide having at least 98% sequence identity to the mature polypeptide of SEQ ID NO: 3.

An embodiment of the invention is a composition comprising an isolated polypeptide having at least 99% sequence identity to the mature polypeptide of SEQ ID NO: 3.

An embodiment of the invention is a composition comprising an isolated polypeptide having 100% sequence identity to the mature polypeptide of SEQ ID NO: 3.

In one aspect, the composition comprises or consists of the mature amino acid sequence of SEQ ID NO: 3 or an allelic variant thereof; or is a fragment thereof having $\alpha$-L-galactosidase activity. In another aspect, the composition comprises or consists of the mature polypeptide of SEQ ID NO: 2. In a further aspect, the composition comprises or consists of the mature polypeptide of SEQ ID NO: 3. In another aspect, the composition comprises or consists of amino acids 1 to 160 of SEQ ID NO: 2, amino acids 5 to 154 of SEQ ID NO: 2, or amino acids 10 to 149 of SEQ ID NO: 2. In another aspect, the polypeptide comprises or consists of amino acids 1 to 160 of SEQ ID NO: 3, amino acids 5 to 154 of SEQ ID NO: 3, or amino acids 10 to 149 of SEQ ID NO: 3.

The present invention also relates to compositions comprising isolated polypeptides having having $\alpha$-L-galactosidase activity that are encoded by polynucleotides that hybridize under high stringency conditions, or very high stringency conditions with (i) the mature polypeptide coding sequence of SEQ ID NO: 1, and/or (ii) the full-length complementary strand of (i) (J. Sambrook, E.F. Fritsch, and T. Maniatis, 1989, Molecular Cloning, A Laboratory Manual, 2d edition, Cold Spring Harbor, New York).

The present invention further relates to compositions comprising isolated polypeptides that differ by no more than one hundred amino acid residues, *e.g.*, by ninety amino acids, by eighty amino acids, by seventy amino acids, by sixty amino acids, by fifty amino acids, by fourty amino acids, by thirty amino acids, by twentyfive amino acids, by twenty amino acids, by fifteen amino acids, by ten amino acids, by eight amino acids, by seven amino acids, by six amino acids, by five amino acids, by four amino acids, by three amino acids, by two amino acids, and by one amino acid from the polypeptide of SEQ ID NO: 3.

The present invention also relates to compositions comprising variants comprising a substitution, deletion, and/or insertion of one or more (or several) amino acids of SEQ ID NO: 3 or a homologous sequence thereof. The total number of positions having amino acid substitutions, deletions and/or insertions in SEQ ID NO: 3 is not more than 100, *e.g.,* 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 40, 50, 60, 70, 80, 90 or 100. Preferably, amino acid changes are of a minor nature, that is conservative amino acid substitutions, insertions or deletions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

The embodiments indicated above in respect of SEQ ID NO:3 apply correspondingly to compositions comprising or consisting of polypeptides as provided by SEQ ID NO:2.

The embodiments indicated above in respect of SEQ ID NO:3 apply correspondingly to compositions comprising or consisting of polypeptides as provided by SEQ ID NO:4.

The embodiments indicated above in respect of SEQ ID NO:3 apply correspondingly to compositions comprising or consisting of polypeptides as provided by SEQ ID NO:5.

The embodiments indicated above in respect of SEQ ID NO:3 apply correspondingly to compositions comprising or consisting of polypeptides as provided by SEQ ID NO:6.

The compositions may comprise $\alpha$-L-galactosidase of the present invention as the major enzymatic component, *e.g.*, a mono-component composition. Alternatively, the compositions may comprise multiple enzymatic activities, such as one or more (*e.g.*, several) enzymes selected from the group consisting of hydrolase, isomerase, ligase, lyase, oxidoreductase, or transferase, *e.g.*, an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

The additional enzyme(s) may be produced, for example, by a microorganism such as bacteria or fungi or by plants or by animals. The compositions may be prepared in accordance with methods known in the art and may be in the form of a liquid or a dry composition. For instance, the composition may be in the form of a granulate or a microgranulate. The $\alpha$-L-galactosidase may be stabilized in accordance with methods known in the art.

The compositions may be prepared in accordance with methods known in the art and may be in the form of a liquid or a dry composition. The compositions may be stabilized in accordance with methods known in the art.

The compositions may comprise a polypeptide of the present invention as the major enzymatic component, *e.g.*, a mono-component composition. Alternatively, the compositions may comprise multiple enzymatic activities, such as one or more (*e.g.*, several) enzymes selected from the group consisting of hydrolase, isomerase, ligase, lyase, oxidoreductase, or transferase, *e.g.*, an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

The compositions may be prepared in accordance with methods known in the art and may be in the form of a liquid or a dry composition. The compositions may be stabilized in accordance with methods known in the art.

Examples are given below of preferred uses of the compositions of the present invention. The dosage of the composition and other conditions under which the composition is used may be determined on the basis of methods known in the art.

**Use of α-L-galactosidases of the Invention in Animal Feed**

The term animal includes all animals. Examples of animals are non-ruminants, and ruminants. Ruminant animals include, for example, animals such as sheep, goats, and cattle, e.g. beef cattle, cows, and young calves. In a particular embodiment, the animal is a non-ruminant animal. Non-ruminant animals include mono-gastric animals, e.g. pigs or swine (including, but not limited to, piglets, growing pigs, and sows); poultry such as turkeys, ducks and chicken (including but not limited to broiler chicks, layers); horses (including but not limited to hotbloods, coldbloods and warm bloods), young calves; and fish (including but not limited to salmon, trout, tilapia, catfish and carps); and crustaceans (including but not limited to shrimps and prawns).

The term feed or feed composition means any compound, preparation, mixture, or composition suitable for, or intended for intake by an animal. In the use according to the invention the α-L-galactosidase can be fed to the animal before, after, or simultaneously with the diet. The latter is preferred.

In a particular embodiment, the α-L-galactosidase, in the form in which it is added to the feed, or when being included in a feed additive, is well-defined. Well-defined means that the □-xylosidase preparation is at least 50% pure as determined by Size-exclusion chromatography (see Example 12 of WO 01/58275). In other particular embodiments the α-L-galactosidase preparation is at least 60, 70, 80, 85, 88, 90, 92, 94, or at least 95% pure as determined by this method.

A well-defined α-L-galactosidase preparation is advantageous. For instance, it is much easier to dose correctly to the feed a □-xylosidase that is essentially free from interfering or contaminating other proteases or other proteins in general. The term dose correctly refers in particular to the objective of obtaining consistent and constant results, and the capability of optimising dosage based upon the desired effect.

For the use in animal feed, however, the α-L-galactosidase need not be that pure; it may e.g. include other enzymes, in which case it could be termed a α-L-galactosidase preparation.

The α-L-galactosidase preparation can be (a) added directly to the feed (or used directly in a protein treatment process), or (b) it can be used in the production of one or more intermediate compositions such as feed additives or premixes that is subsequently added to the feed (or used in a treatment process). The degree of purity described above refers to the purity of the original α-L-galactosidase preparation, whether used according to (a) or (b) above.

α-L-galactosidase preparations with purities of this order of magnitude are in particular obtainable using recombinant methods of production, whereas they are not so easily obtained and also subject to a much higher batch-to-batch variation when the α-L-galactosidase is produced by traditional fermentation methods. Such α-L-galactosidase preparation may of course be mixed with other enzymes to obtain a preparation with two or more purified enzymes with different or similar activities.

The substrate protein may be an animal protein, such as meat and bone meal, feather meal, and/or fish meal; or it may be a vegetable protein.

The substrate source is preferentially of vegetable origin. The term vegetable or vegetable protein as used herein refers to any compound, composition, preparation or mixture that includes at least one protein derived from or originating from a vegetable, including modified proteins and protein-derivatives. In particular embodiments, the protein content of the vegetable proteins is at least 10, 20, 30, 40, 50 or 60 % (w/w).

Vegetable proteins may be derived from vegetable sources containing galactose cell wall constituents such as legumes and cereals (Bach Knudsen, K. E. (1997). Carbohydrate and lignin contents of plant materials used in animal feeding. Animal Feed Science and Technology 67: 319-338), for example materials from plants of the families Fabaceae (Leguminosae), Cruciferaceae, Chenopodiaceae, and Poaceae, such as soy bean meal, lupin meal and rapeseed meal. In a particular embodiment, the vegetable protein source is material from one or more plants of the family Fabaceae, e.g. soybean, lupine, pea, or bean. In another particular embodiment, the vegetable protein source is material from one or more plants of the family Chenopodiaceae, e.g. beet, sugar beet, spinach or quinoa. Other examples of vegetable protein sources are rapeseed, sunflower seed, cotton seed, and cabbage. Soybean is a preferred vegetable protein source. Other examples of vegetable protein sources are cereals such as barley, wheat, rye, oat, maize (corn), rice, triticale, sorghum, dried distillers grains with solubles (DDGS) and microalgae.

In a particular embodiment of a treatment process the α-L-galactosidase(s) in question is affecting (or acting on, or exerting its hydrolyzing or degrading influence on) the proteins, such as vegetable proteins or protein sources. To achieve this, the protein or protein source is typically suspended in a solvent, e.g. an aqueous solvent such as water, and the pH and temperature values are adjusted paying due regard to the characteristics of the enzyme in question. For example, the treatment may take place at a pH-value at which the activity of the actual α-L-galactosidase is at least 5%, 10%, 20%, 30%, 40%, 50%, 60%, 70%, 80%, or at least 90%. Likewise, for example, the treatment may take place at a temperature at which the activity of the actual α-L-galactosidase is at least 5%, 10%, 20%, 30%, 40%, 50%, 60%, 70%, 80%, or at least 90%. The above percentage activity indications are relative to the maximum activities. The enzymatic reaction is continued until the desired result is achieved, following which it may or may not be stopped by inactivating the enzyme, e.g. by a heat-treatment step.

In another particular embodiment of a treatment process of the invention, α-L-galactosidase is added to the vegetable protein source, but its hydrolysing influence is not activated until later when desired. Once suitable hydrolysing conditions are established, or once any enzyme inhibitors are inactivated, or whatever other means could have been applied to postpone the action of the enzyme.

In one embodiment, the treatment is a pre-treatment of animal feed or proteins for use in animal feed, i.e. the vegetable protein sources are hydrolysed before intake.

The term improving the nutritional value of an animal feed means improving the availability of nutrients in the feed. In this invention improving the nutritional values refers in particular to improving the availability of the protein fraction of the feed, thereby leading to increased protein extraction, higher protein yields, and/or improved protein utilization. When the nutritional value of the feed is increased, the protein and/or amino acid digestibility is increased and the growth rate and/or weight gain and/or feed conversion (i.e. the weight of ingested feed relative to weight gain) of the animal might be improved.

The α-L-galactosidase can be added to the feed in any form, be it as a relatively pure α-xylosidase, or in admixture with other components intended for addition to animal feed, i.e. in the form of animal feed additives, such as the so-called pre-mixes for animal feed.

In a further aspect, the present invention relates to compositions for use in animal feed, such as animal feed, and animal feed additives, e.g. premixes.

Apart from the α-L-galactosidase of the invention, the animal feed additives of the invention contain at least one fat-soluble vitamin, and/or at least one water soluble vitamin, and/or at least one trace mineral, and/or at least one macro mineral.

Further, optional, feed-additive ingredients are colouring agents, e.g. carotenoids such as beta-carotene, astaxanthin, and lutein; stabilisers; growth improving additives and aroma compounds/flavourings, e.g. creosol, anethol, deca-, undeca-and/or dodeca-lactones, ionones, irone, gingerol, piperidine, propylidene phatalide, butylidene phatalide, capsaicin and/or tannin; antimicrobial peptides; polyunsaturated fatty acids (PUFAs); reactive oxygen generating species; also, a support may be used that may contain, for example, 40-50% by weight of wood fibres, 8-10% by weight of stearine, 4-5% by weight of curcuma powder, 4-58% by weight of rosemary powder, 22-28% by weight of limestone, 1-3% by weight of a gum, such as gum arabic, 5-50% by weight of sugar and/or starch and 5-15% by weight of water.

A feed or a feed additive of the invention may also comprise at least one other enzyme selected from amongst phytase (EC 3.1.3.8 or 3.1.3.26); xylanase (EC 3.2.1.8); galactanase (EC 3.2.1.89); alpha-galactosidase (EC 3.2.1.22); protease (EC 3.4), phospholipase A1 (EC 3.1.1.32); phospholipase A2 (EC 3.1.1.4); lysophospholipase (EC 3.1.1.5); phospholipase C (3.1.4.3); phospholipase D (EC 3.1.4.4); amylase such as, for example, alpha-amylase (EC 3.2.1.1); and/or beta-glucanase (EC 3.2.1.4 or EC 3.2.1.6).

In a particular embodiment, the feed or a feed additive of the invention also comprises a protease (EC 3.4).

In a particular embodiment, the feed or a feed additive of the invention also comprises a phytase (EC 3.1.3.8 or 3.1.3.26).

In a particular embodiment, the feed or a feed additive of the invention also comprises a xylanase (EC 3.2.1.8).

A feed or a feed additive of the invention may also comprise at least one probiotic or direct fed microbial (DFM) optionally together with one or more other enzymes selected from amongst phytase (EC 3.1.3.8 or 3.1.3.26); xylanase (EC 3.2.1.8); galactanase (EC 3.2.1.89); alpha-galactosidase (EC 3.2.1.22); protease (EC 3.4), phospholipase A1 (EC 3.1.1.32); phospholipase A2 (EC 3.1.1.4); lysophospholipase (EC 3.1.1.5); phospholipase C (3.1.4.3); phospholipase D (EC 3.1.4.4); amylase such as, for example, alpha-amylase (EC 3.2.1.1); and/or beta-glucanase (EC 3.2.1.4 or EC 3.2.1.6).

The DFM may be added to the animal feed such that the daily dose of DFM is between 1x10$^5$ CFU and 1x10$^{13}$ CFU, preferably between 1x10$^6$ CFU and 1x10$^{12}$ CFU, more preferably between 1x10$^7$ CFU and 1x10$^{11}$ CFU and even more preferably between 5x10$^7$ CFU and 1x10$^{10}$ CFU. Alternatively, the DFM may be added to the animal feed such that the concentration of DFM in the feed is between 1x10$^3$ CFU/g feed and 1x10$^8$ CFU/g feed, preferably between 5x10$^3$ CFU/g feed and 1x10$^7$ CFU/g feed, more preferably between 1x10$^4$ CFU/g feed and 5x10$^6$ CFU/g feed and even more preferably between 2.5x10$^4$ CFU/g feed and 1x10$^6$ CFU/g feed.

The direct fed microbial may be a bacterium from one or more of the following genera: Lactobacillus, Lactococcus, Streptococcus, Bacillus, Pediococcus, Enterococcus, Leuconostoc, Carnobacterium, Propionibacterium, Bifidobacterium, Clostridium and Megasphaera or any combination thereof, preferably from Bacillus subtilis, Bacillus licheniformis, Bacillus amyloliquefaciens, Enterococcus faecium, Enterococcus spp, and Pediococcus spp, Lactobacillus spp, Bifidobacterium spp, Lactobacillus acidophilus, Pediococsus acidilactici, Lactococcus lactis, Bifidobacterium bifidum, Propionibacterium thoenii, Lactobacillus farciminus, lactobacillus rhamnosus, Clostridium butyricum, Bifidobacterium animalis ssp. animalis, Lactobacillus reuteri, Bacillus cereus, Lactobacillus salivarius ssp. salivarius, Megasphaera elsdenii, Propionibacteria sp and more preferably from Bacillus subtilis strains 3A-P4 (PTA-6506); 15A-P4 (PTA-6507); 22C-P1 (PTA-6508); 2084 (NRRL B-500130); LSSA01 (NRRL-B-50104); BS27 (NRRL B-501 05); BS 18 (NRRL B-50633); and BS 278 (NRRL B-50634).

In a particular embodiment, these other enzymes are well-defined (as defined above for In a particular embodiment, the feed or a feed additive of the invention also comprises a xylanase (EC 3.2.1.8).

Examples of antimicrobial peptides (AMP's) are CAP18, Leucocin A, Tritrpticin, Protegrin-1, Thanatin, Defensin, Lactoferrin, Lactoferricin, and Ovispirin such as Novispirin (Robert Lehrer, 2000), Plectasins, and Statins, including the compounds and polypeptides disclosed in WO 03/044049 and WO 03/048148, as well as variants or fragments of the above that retain antimicrobial activity.

Examples of antifungal polypeptides (AFP's) are the Aspergillus giganteus, and Aspergillus niger peptides, as well as variants and fragments thereof which retain antifungal activity, as disclosed in WO 94/01459 and WO 02/090384.

Examples of polyunsaturated fatty acids are C18, C20 and C22 polyunsaturated fatty acids, such as arachidonic acid, docosohexaenoic acid, eicosapentaenoic acid and gamma-linoleic acid.

Examples of reactive oxygen generating species are chemicals such as perborate, persulphate, or percarbonate; and enzymes such as an oxidase, an oxygenase or a syntethase.

Usually fat- and water-soluble vitamins, as well as trace minerals form part of a so-called premix intended for addition to the feed, whereas macro minerals are usually separately added to the feed. Either of these composition types, when enriched with a □-xylosidase of the invention, is an animal feed additive of the invention.

In a particular embodiment, the animal feed additive of the invention is intended for being included (or prescribed as having to be included) in animal diets or feed at levels of 0.01 to 10.0%; more particularly 0.05 to 5.0%; or 0.2 to 1.0% (% meaning g additive per 100 g feed). This is so in particular for premixes.

The following are non-exclusive lists of examples of these components:

Examples of fat-soluble vitamins are vitamin A, vitamin D3, vitamin E, and vitamin K, e.g. vitamin K3.

Examples of water-soluble vitamins are vitamin B12, biotin and choline, vitamin B1, vitamin B2, vitamin B6, niacin, folic acid and panthothenate, e.g. Ca-D-panthothenate.

Examples of trace minerals are manganese, zinc, iron, copper, iodine, selenium, and cobalt.

Examples of macro minerals are calcium, phosphorus and sodium.

The nutritional requirements of these components (exemplified with poultry and piglets/pigs) are listed in Table A of WO 01/58275. Nutritional requirement means that these components should be provided in the diet in the concentrations indicated.

In the alternative, the animal feed additive of the invention comprises at least one of the individual components specified in Table A of WO 01/58275. At least one means either of, one or more of, one, or two, or three, or four and so forth up to all thirteen, or up to all fifteen individual components. More specifically, this at least one individual component is included in the additive of the invention in such an amount as to provide an in-feed-concentration within the range indicated in column four, or column five, or column six of Table A.

In a still further embodiment, the animal feed additive of the invention comprises at least one of the below vitamins, preferably to provide an in-feed-concentration within the ranges specified in the below Table 1 (for piglet diets, and broiler diets, respectively).

Table 1: Typical vitamin recommendations

| Vitamin | Piglet diet | Broiler diet |
|---|---|---|
| Vitamin A | 10,000-15,000 IU/kg feed | 8-12,500 IU/kg feed |
| Vitamin D3 | 1800-2000 IU/kg feed | 3000-5000 IU/kg feed |
| Vitamin E | 60-100 mg/kg feed | 150-240 mg/kg feed |
| Vitamin K3 | 2-4 mg/kg feed | 2-4 mg/kg feed |
| Vitamin B1 | 2-4 mg/kg feed | 2-3 mg/kg feed |
| Vitamin B2 | 6-10 mg/kg feed | 7-9 mg/kg feed |
| Vitamin B6 | 4-8 mg/kg feed | 3-6 mg/kg feed |
| Vitamin B12 | 0.03-0.05 mg/kg feed | 0.015-0.04 mg/kg feed |
| Niacin (Vitamin B3) | 30-50 mg/kg feed | 50-80 mg/kg feed |
| Pantothenic acid | 20-40 mg/kg feed | 10-18 mg/kg feed |

(continued)

| Vitamin | Piglet diet | Broiler diet |
|---|---|---|
| Folic acid | 1-2 mg/kg feed | 1-2 mg/kg feed |
| Biotin | 0.15-0.4 mg/kg feed | 0.15-0.3 mg/kg feed |
| Choline chloride | 200-400 mg/kg feed | 300-600 mg/kg feed |

The present invention also relates to animal feed compositions. Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore such fish diets usually have a crude fat content of 200-310 g/kg. WO 01/58275 corresponds to US 09/779334.

An animal feed composition according to the invention has a crude protein content of 50-800 g/kg, and furthermore comprises at least one $\alpha$-L-galactosidase as claimed herein.

Furthermore, or in the alternative (to the crude protein content indicated above), the animal feed composition of the invention has a content of metabolisable energy of 10-30 MJ/kg; and/or a content of calcium of 0.1-200 g/kg; and/or a content of available phosphorus of 0.1-200 g/kg; and/or a content of methionine of 0.1-100 g/kg; and/or a content of methionine plus cysteine of 0.1-150 g/kg; and/or a content of lysine of 0.5-50 g/kg.

In particular embodiments, the content of metabolisable energy, crude protein, calcium, phosphorus, methionine, methionine plus cysteine, and/or lysine is within any one of ranges 2, 3, 4 or 5 in Table B of WO 01/58275 (R. 2-5).

Crude protein is calculated as nitrogen (N) multiplied by a factor 6.25, i.e. Crude protein (g/kg)= N (g/kg) x 6.25. The nitrogen content is determined by the Kjeldahl method (A.O.A.C., 1984, Official Methods of Analysis 14th ed., Association of Official Analytical Chemists, Washington DC).

Metabolisable energy can be calculated on the basis of the NRC publication Nutrient requirements in swine, ninth revised edition 1988, subcommittee on swine nutrition, committee on animal nutrition, board of agriculture, national research council. National Academy Press, Washington, D.C., pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.

The dietary content of calcium, available phosphorus and amino acids in complete animal diets is calculated on the basis of feed tables such as Veevoedertabel 1997, gegevens over chemische samenstelling, verteerbaarheid en voederwaarde van voedermiddelen, Central Veevoederbureau, Runderweg 6, 8219 pk Lelystad. ISBN 90-72839-13-7.

In a particular embodiment, the animal feed composition of the invention contains at least one vegetable protein as defined above.

The animal feed composition of the invention may also contain animal protein, such as Meat and Bone Meal, Feather meal, and/or Fish Meal, typically in an amount of 0-25%. The animal feed composition of the invention may also comprise Dried Distillers Grains with Solubles (DDGS), typically in amounts of 0-30%.

In still further particular embodiments, the animal feed composition of the invention contains 0-80% maize; and/or 0-80% sorghum; and/or 0-70% wheat; and/or 0-70% barley; and/or 0-30% oats; and/or 0-40% soybean meal; and/or 0-25% fish meal; and/or 0-25% meat and bone meal; and/or 0-20% whey.

Animal diets can e.g. be manufactured as mash feed (non-pelleted) or pelleted feed. Typically, the milled feed-stuffs are mixed and sufficient amounts of essential vitamins and minerals are added according to the specifications for the species in question. Enzymes can be added as solid or liquid enzyme formulations. For example, for mash feed a solid or liquid enzyme formulation may be added before or during the ingredient mixing step. For pelleted feed the (liquid or solid) □-xylosidase /enzyme preparation may also be added before or during the feed ingredient step. Typically a liquid □-xylosidase /enzyme preparation is added after the pelleting step. The enzyme may also be incorporated in a feed additive or premix.

The final enzyme concentration in the diet is within the range of 0.01-200 mg enzyme protein per kg diet, for example in the range of 0.5-25 mg enzyme protein per kg animal diet.

The $\alpha$-L-galactosidase should of course be applied in an effective amount, i.e. in an amount adequate for improving protein hydrolysis, protein and amino acid digestibility, and/or improving nutritional value of feed. It is at present contemplated that the enzyme is administered in one or more of the following amounts (dosage ranges): 0.01-200; 0.01-100; 0.5-100; 1-50; 5-100; 10-100; 0.05-50; or 0.10-10 - all these ranges being in mg □-xylosidase protein per kg feed (ppm).

For determining mg $\alpha$-L-galactosidase protein per kg feed, the $\alpha$-L-galactosidase is purified from the feed composition, and the specific activity of the purified $\alpha$-L-galactosidase is determined using a relevant assay (see under $\alpha$-L-galactosidase activity, substrates, and assays). The $\alpha$-L-galactosidase activity of the feed composition as such is also determined using the same assay, and on the basis of these two determinations, the dosage in mg $\alpha$-L-galactosidase

protein per kg feed is calculated.

The same principles apply for determining mg α-L-galactosidase protein in feed additives. Of course, if a sample is available of the α-L-galactosidase used for preparing the feed additive or the feed, the specific activity is determined from this sample (no need to purify the α-L-galactosidase from the feed composition or the additive).

**Signal Peptide**

Here disclosed is an isolated polynucleotide encoding a signal peptide comprising or consisting of amino acids 1 to 19 of SEQ ID NO: 2. The polynucleotides may further comprise a gene encoding a protein, which is operably linked to the signal peptide. The protein is preferably foreign to the signal peptide. In one aspect, the polynucleotide encoding the signal peptide is nucleotides 1 to 57 of SEQ ID NO: 1.

**Nucleic Acid Constructs, Expression Vectors, Recombinant Host Cells, and Methods for Production of α-L-galactosidase**

Disclosed are nucleic acid constructs, expression vectors and recombinant host cells comprising such polynucleotides. Also disclosed are methods of producing a protein, comprising (a) cultivating a recombinant host cell comprising such polynucleotide; and (b) recovering the protein.

The protein may be native or heterologous to a host cell. The term "protein" is not meant herein to refer to a specific length of the encoded product and, therefore, encompasses peptides, oligopeptides, and polypeptides. The term "protein" also encompasses two or more polypeptides combined to form the encoded product. The proteins also include hybrid polypeptides and fused polypeptides.

Preferably, the protein is a hormone, enzyme, receptor or portion thereof, antibody or portion thereof, or reporter. For example, the protein may be a hydrolase, isomerase, ligase, lyase, oxidoreductase, or transferase, *e.g.*, an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

Preferably the protein is a α-L-galactosidase.

The gene may be obtained from any prokaryotic, eukaryotic, or other source.

The present invention is further described by the following examples that should not be construed as limiting the scope of the invention.

**Examples**

**Strains**

Reference has been made herein to the publicly available deposit of the microbial strain Bacteroides ovatus (ATCC 8483). deposited with the ATCC in 1933 by Eggerth (Eggerth AH, Gagnon BH. The bacteroides of human feces. J. Bacteriol. 25: 389-413, 1933).

**Example 1:**

**Construction of plasmids and protein production**

To produce protein constructs DNA sequences encoding *Bacteroides ovatus* GH31 (BACOVA 03422; accession A7LZZ5) and GH95 (BACOVA_03438; accession A7M011) were amplified by PCR from *B. ovatus* ATCC8483 genomic DNA using suitable primers. The amplified DNA fragment encoding the GH95 was cloned into Ndel and Xhol restricted pET21a (Novagen).

Soluble recombinant proteins were produced by inoculating recombinant E. coli (BL21; Novagen) into LB medium (1 L) in 2 L flasks supplemented with ampicillin (50 μg/mL) and incubating the cultures at 37 °C with shaking (200rpm) until the OD600 reached 0.4. Isopropyl β-D-thiogalactopyranoside was added to final concentration of 1 mM to induce protein production, and the culture was incubated for an additional 5 h at 37 °C. Cells were harvested by centrifugation (fixed angle) at 5000g/10min/4 °C, resuspended in 10 mL of 20 mM Tris-HCl, pH 8.0, containing 300 mM NaCl (buffer A) per litre of culture and disrupted by sonication. Following clarification of the cell lysate by centrifugation at 15000g/30 min/4 °C, the polypeptide was purified by immobilized metal affinity chromatography using Talon resin (Clontech). Proteins were eluted with buffer A containing 150mM imidazole. *B. ovatus* GH95 was dialysed against 50mM Na2PO4 buffer, pH 7.5.

The cloned DNA sequence of BACOVA_03438 GH95 is provide as SEQ ID NO:1 and the deduced amino acid sequence as SEQ ID NO:2. The mature amino acid sequence is provided as SEQ ID NO:3

**Example 2:**

**Biochemical characterisation of BACOVA_03438 GH95**

**Sources of carbohydrates used:**

Wheat and rye arabinoxylans and xylo-oligosaccharides were from Megazyme (Megazyme International Ireland). Birch and Oat Spelt xylans and all monosaccharides were from Sigma. Corn bran xylan was a kind gift from Drs Kevin Hicks and Madhav Yadav (USDA).

**Thin Layer Chromatography (TLC):**

Activity of the GH95 enzyme against a range of decorated xylans was initially assessed by TLC. Xylans (1% w/v final) from rye, wheat, birch, oat and corn bran were incubated with 0.5 $\mu$M of each enzyme. Reactions were carried out in 50mM K2PO4, 120mM citric acid pH 6.5. Polysaccharides where incubated with enzymes for 5h at 37°C. Then samples (6$\mu$l each) were loaded at 1cm intervals on the TLC plate and dried using a hair drier after each loading of 2ul. The TLC plate was then placed in 1L glass tank containing running buffer (butanol/acetic acid/water at 2:2:1), to a depth 0.5 cm and sealed with glass plate. When the running buffer reached ~1cm from the top of the plate, the plate was taken out dried with hair drier and immersed for a few seconds in an orcinol sulphuric acid reagent (sulphuric acid/ethanol/water 3:70:20 v/v, orcinol 1%), and dried at 120°C until sugars were revealed (~5-10min).

**High performance liquid chromatography (HPLC):**

HPLC was used to quantify the products of corn xylan hydrolysis by both the GH31 and GH95 enzymes, using an analytical CARBOPAC™ PA-100 anion exchange column (Dionex) equipped with a CARBOPAC™ PA-100 guard column. The fully automated system had a loop size of 100 $\mu$l, flow rate of 1.0 ml/min, pressure of -2000 psi and sugars were detected by pulsed amperometric detection (PAD). The PAD settings were E1 = +0.05, E2 = +0.6 and E3 = - 0.6. The elution conditions used were 0-5 minutes 100 mM NaOH, 5-15 min 100 mM NaOH with 0-75mM sodium acetate gradient and 15-25 min 100mM NaOH containing 75mM sodium acetate. Before and after each run the column was washed with 500 mM sodium acetate for 10 min and then 500 mM sodium hydroxide for 5 min and then equilibrated with 100 mM sodium hydroxide for 10 min.

Enzyme reactions were performed in 50 mM PC buffer, pH 6.5 containing 1 mg/ml BSA. The final reaction volume was calculated to allow 4-8 aliquots to be analysed from each reaction, which was started by addition of suitably diluted enzyme (1/100th of final volume). Reactions were terminated by boiling aliquots for 10 min. Data were collected and analysed using XChrom V.2.04 Software (LabSystems) via a VG Chromatography Server (Fisons Instruments).

**Example 3:**

**Activity of *B. ovatus* GH95, BACOVA_03438.**

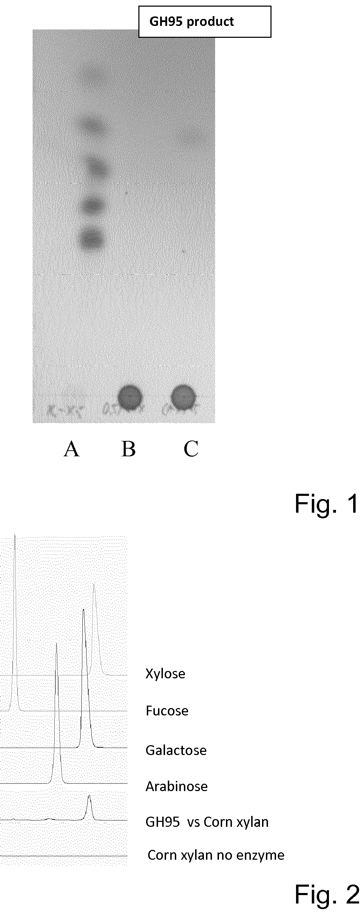

TLC analysis revealed that BACOVA_03438 GH95 was not active against decorated xylans from wheat, rye, oat spelt or birchwood (data not shown) but that the enzyme released a single product from corn bran xylan that co-migrated with L-galactose (Figure 1).

HPLC was used to investigate further the identity of the B. ovatus GH95 product released from corn bran xylan. The data revealed a single product liberated by B. ovatus GH95 from corn bran xylan (Figure 2). All characterized enzymes within GH95 are $\alpha$-L-fucosidases, but the GH95 product does not co-elute with L-fucose by HPLC (Figure 10). L-fucose is a hexose deoxy sugar which lacks of a hydroxyl group on the carbon at the 6-position (C-6) and it is equivalent to 6-deoxy-L-galactose. The product of GH95 activity on corn bran xylan co-elutes with L-galactose on the HPLC, indicating the enzyme is an $\alpha$-L-galactosidase, a novel enzyme activity.

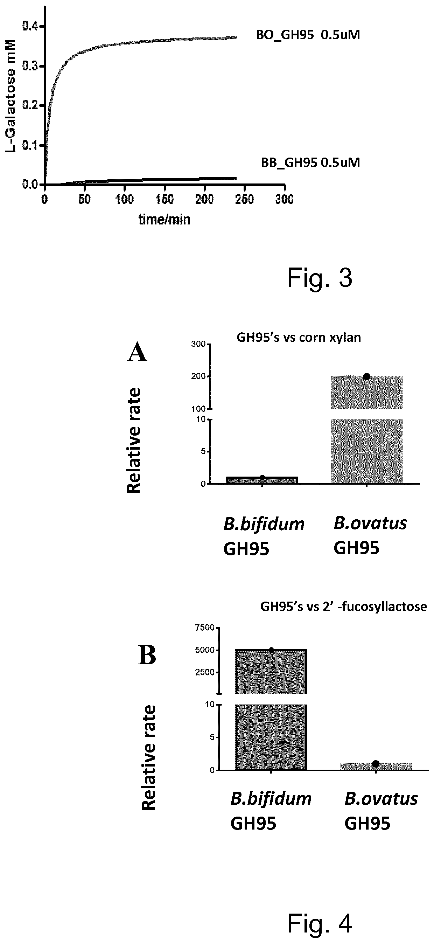

We then compared the activity of the *B. ovatus* enzyme GH95 (BACOVA_03438) with a previously characterized GH95 $\alpha$-L-fucosidase from *Bifidobacterium bifidum* (AAQ72464). The data (Figures 3 and 4) showed that although *B. bifidum* enzyme is able to liberate galactose from corn bran xylan, the rate of the B. ovatus GH95 on the same amount of polymeric substrate was at least 200 times greater. It is clear from these data that $\alpha$-L-galactosidase activity is not a general property of GH95 enzymes.

**Example 5:**

**Corn destarching and Corn fiber gum (CFG) extraction**

Destarched corn

107 kg pre-milled corn (<1.0 mm) mixed with 253 kg water at 53 °C. The mixture was heated to 95 °C and the pH adjusted to 6.2 with 1 M NaOH. 1.12 kg, Termamyl 120 L was added and the reaction was kept around 90 °C for 3 hrs. After 3 hrs, cold water was added to a total reaction weight of 600 kg. Liquid solid separation was done with a Westfalia decanter, CA-225-110, 4950 rpm, flow 600 L/hour. The solid fiber fraction was re-slurried and separated twice with water to a total weight of 600 kg followed by separation as described above. The final fiber fraction was divided into smaller portions and freeze dried for 3-4 days.

Corn Fiber Gum extraction

20 g of destarched corn was added to 200 mL boiling MilliQ™ water together with 0.8 g of NaOH and 0.8 g of Ca(OH)2. The mixture was kept for 1 hour at 96 C and then centrifuged for 20 min at 6000 g.
The supernatant looked milky and fatty and therefore fats were extracted by shaking with hexane (1 part hexane to 4 parts corn fiber gum). After a resting time the hexane was removed by pipetting.
A 2nd extraction (CFG2) was made by dissolving the pellet in 200 mL 1 M NaOH. The mixture was kept for 1 hour at 96 C and then centrifuged for 20 min at 6000 g and the supernatant adjusted to pH 6 with 4 M HCl.
Both CFG fractions were concentrated using a rotavap and precipitated in EtOH at a final concentration of 90 %. The pellet from the 0.1 M NaOH will from now on be called CFG1. Due to the high pH in 1 M NaOH extract (CFG2) this fraction was dialysed on a 2 L measuring cylinder with deionised water with the tap dripping overnight using a 3 kDa dialysis membrane.
Before application trials both CFG1 and CFG2 were centrifuged at 25000 g for 25 min and filtered through a 0.44 $\mu$m syringe filter. The dry matter in CFG2 fractions was determined with a Mettler Toledo HR73.

**Enzymatic corn fiber gum hydrolysis**

The enzymatic hydrolysis was performed using the CFG2 fraction (vide supra).
The 400 $\mu$L assays were run at 40°C on a shaking Eppendorph Thermomixer, 1200 rpm for 17 hrs and the reactions were stopped by heat at 97°C for 10 min. The assay setup can be found in Table 1. Seven different reactions were run in parallel and the enzymes in the respective samples can be found in Table 2.

Table 1.

|  | $\mu$L |
| --- | --- |
| CFG2 2.3 % DM | 350 |
| 0.25 M Na citrate pH6 | 20 |
| Enzyme[a] | 10 |
| MilliQ | to 400 |
| [a]10 $\mu$L per enzyme. Enzyme dosage: GH31, 0.7 g/L; GH95, 0.7 g/L; UltraFlo L 8 % (v/v) | |

Table 2.

| Sample | Enzymes in samples |
| --- | --- |
| 1 | GH31 |
| 2 | GH95 |
| 3 | GH31 + GH95 |
| 4 | UltraFlo L + GH31 |
| 5 | UltraFlo L + GH95 |

(continued)

| Sample | Enzymes in samples |
|--------|---------------------|
| 6 | UltraFlo L + GH31 + GH95 |
| 7 | UltraFlo L |

**HPLC analysis of produced monosaccharides**

The supernatants were analysed on a Dionex ICS-5000 by high performance anion exchange chromatography with pulsed amperometric detection (HPAEC-PAD) using a CarboPac-20 guard and analytical column. The system was controlled by Chromeleon v. 6.8. Released monosaccharides were quantified against a 6-point standard curve of arabinose, galactose, glucose and xylose from 0.0002-0.02 g/L. 25 $\mu$L were injected of each sample using the program in Table 3. The eluents were degassed by bubbling with helium for 10 min and had the following composition: A, MilliQ water; B, 0.5 M NaOH; C, 0.5 M NaOAc; D, 60 mM NaOAc. Sample 1-3 were diluted 1:50 and samples 4-7 were diluted 1:200, both in water, before injection.

Table 3. PA-20 eluent program with a flow of 0.5 mL/min.

| | Eluent (%) | | | | |
|--------|------|------|-------|-----|-------|
| min | **A** | **B** | **C** | **D** | **Curve** |
| 0-8.5 | 80 | 0 | 0 | 20 | 5 |
| 8.5-25 | 80-50 | 0-30 | 20 | 0 | 4 |
| 25-27 | 0-100 | 0 | 100-0 | 0 | 9 |
| 27-40 | 80 | 0 | 0 | 20 | 5 |

GH31 released xylose from corn fiber gum and GH95 released L-galactose. Both GH31 and GH95 seem to add to the hydrolysis achieved by UltraFlo L. Furthermore the GH95 activity is clearly missing from UltraFlo L since no galactose is released in the absence of GH95 (Figure 1).

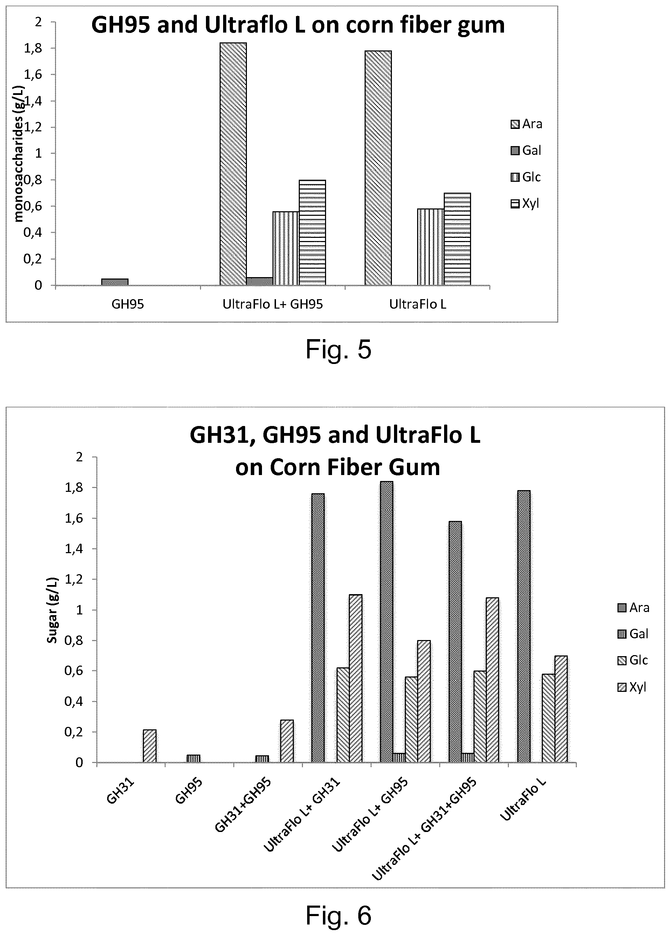

The data in Figures 5 and 6 clearly demonstrate that both GH31 and GH95 could be used to aid in the degradation of corn xylan as well as releasing specifc monosaccharides from corn xylan.

**Example 6:**

**Materials and Methods**

*Substrates*

a. Milled maize (kernels milled to pass a 0.5 mm screen)

b. Industrially prepared de-starched maize (IPDM)-Milled maize was treated with Termamyl for 1.25 hours at 92-95°C and then centrifuged. The residue was washed 3 times with tap water (Temp 13 °C) and finally freeze dried before use. From a 107 kg of starting material we obtained 64.5 kg (before freeze drying) resulting in approximately 20 kg air dry material.

c. NaOH treated IPDM: IDPM was stirred with 0.2 M NaOH at room temperature for 6 h. The pH was adjusted to 5.5 after the 6 h and and washed 5 times with tap water, before being dried with acetone in the lab. From previous studies it has been ascertained that this NaOH treatment of maize removes the acetic and ferulic acid from milled maize.

*Methods and Protocols*

Samples were incubated in glass test tubes at 40°C for 4 h, using NaAc buffer (pH 5). They were centrifuged supernatant was collected for xylose assay (determination of soluble xylan as xylose) for samples h-m (Table 4). Small aliquots of the supernatants were analysed for monosaccharide and oligosaccharide content using a Dionex implemented method (Table 4 samples h-m)

Residue of samples from a-g (Table 4) were analysed for monosaccharides using the NSP method for insoluble fiber.

200 mg De-starched corn (DC) FFS-2013-00063 / Alkali treated de-starched corn FFS-2013-00064

*Conditions*

- 4.9 / 4.8 ml NaAc-buffer incl Ca$^{++}$ pH 5
- 100 / 200 ul Enzyme
- 4 hours at 40°C

Table 4.

| | Treatment | Milled Maize | Industrially prepared de-starched maize (IPDM) | NaOH treated IPDM | NSP | Xylose/Dionex |
|---|---|---|---|---|---|---|
| a | Control | X | x | X | x | |
| b | GH10+GH11 | X | | | x | |
| c | GH10+GH11+GH43+GH51 | X | | | x | |
| d | GH10+GH11+GH43+GH51+GH31 | X | | | x | |
| e | GH10+GH11+GH43+GH51+GH95 | X | | | x | |
| f | GH10+GH11+ GH43+GH51+GH31+GH95 | X | | | x | |
| g | GH10+GH11+GH43+GH51+GH31+ GH95+Ultraflo | X | | | x | |
| h | GH31 | | x | X | | x |
| i | GH95 | | x | X | | x |
| j | GH11+GH43+GH51 | | x | X | | x |
| k | GH11+GH43+GH51+GH31 | | x | X | | x |
| l | GH11+GH43+GH51+GH95 | | x | X | | x |
| m | GH11+GH43+GH51+GH31+GH95 | | x | X | | x |

Table 5. Effect of GH95 on released monosaccharides from destarched corn bran. Sugar levels are expressed as area under curve times a dilution factor.

| | Gal | Xyl |
|---|---|---|
| | Area*dilution factor | |
| Blank | 1 | 0 |
| GH95 | 6 | 0 |
| GH11, 43, 51 | 9 | 23 |
| GH11, 43, 51, 95 | 32 | 34 |

**Biological Material**

Reference has been made herein to the publicly available deposit of the microbial strain *Bacteroides ovatus* (ATCC 8483). deposited with the ATCC in 1933 by Eggerth (Eggerth AH, Gagnon BH. The bacteroides of human feces. J. Bacteriol. 25: 389-413, 1933).

The invention described and claimed herein is not to be limited in scope by the specific aspects herein disclosed, since these aspects are intended as illustrations of several aspects of the invention. Indeed, various modifications of the invention in addition to those shown and described herein will become apparent to those skilled in the art from the foregoing description. In the case of conflict, the present disclosure including definitions will control.

**Claims**

1. Use of an isolated polypeptide having α-L-galactosidase activity, selected from the group consisting of:

    (a) a polypeptide having at least 80%, e.g. at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide of SEQ ID NO: 2 or SEQ ID NO:3;
    (b) a polypeptide encoded by a polynucleotide that hybridizes under high stringency conditions or very high stringency conditions with (i) the mature polypeptide coding sequence of SEQ ID NO: 1, or (ii) the full-length complement of (i);
    (c) a polypeptide encoded by a polynucleotide at least 80%, e.g. at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the mature polypeptide coding sequence of SEQ ID NO: 1
    (d) a variant of the mature polypeptide of SEQ ID NO: 2 or SEQ ID NO: 3 comprising a substitution, deletion, and/or insertion at one or more positions, wherein the polypeptides differ by no more than 10 amino acids; and
    (e) a fragment of the polypeptide of (a), (b), (c), or (d) that has α-L-galactosidase activity wherein the fragment contains at least 90% of the amino acids of the mature peptide,

    in animal feed.

2. The use according to claim 1, wherein the polypeptide comprises or consists of SEQ ID NO: 2, SEQ ID NO: 3, SEQ ID NO: 4, SEQ ID NO: 5 or SEQ ID NO: 6 or the mature polypeptide of SEQ ID NO: 2, SEQ ID NO: 4 SEQ ID NO:5 or SEQ ID NO: 6.

3. The use according to any of claims 1 to 2, wherein the mature polypeptide is amino acids 20 to 811 of SEQ ID NO: 2, SEQ ID NO: 3, SEQ ID NO: 4, SEQ ID NO: 5 or SEQ ID NO: 6.

4. A composition comprising an isolated polypeptide having α-L-galactosidase activity, selected from the group consisting of:

    (a) a polypeptide having at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 2, SEQ ID NO: 3, SEQ ID NO: 4, SEQ ID NO: 5 or SEQ ID NO: 6;
    (b) a polypeptide encoded by a polynucleotide that hybridizes under high stringency conditions or very-high stringency conditions with:

        (i) the mature polypeptide coding sequence of SEQ ID NO: 1; and/or
        (ii) the full-length complementary strand of (i);

    (c) a polypeptide encoded by a polynucleotide having at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 2, SEQ ID NO:3, SEQ ID NO: 4, SEQ ID NO: 5 or SEQ ID NO: 6;
    (d) a variant comprising a substitution, deletion, and/or insertion of one or more (several) amino acids of SEQ ID NO: 3, wherein the polypeptides differ by no more than 30 amino acids; and
    (e) a fragment of a polypeptide of (a), (b), (c) or (d), that has α-L-galactosidase activity wherein the fragment contains at least 90% of the amino acids of the mature peptide.

**5.** The composition of claim 4, wherein the polypeptide comprises or consists of SEQ ID NO: 2, SEQ ID NO: 3, SEQ ID NO: 4, SEQ ID NO: 5 or SEQ ID NO: 6 or the mature polypeptide of SEQ ID NO: 2, SEQ ID NO: 4 SEQ ID NO:5 or SEQ ID NO: 6.

**6.** The composition of any of claims 4 to 5 that is in the form of a granulate or a microgranulate.

**7.** A whole broth formulation or cell culture composition comprising a polypeptide indicated in any of claims 1-5.

**8.** Use of at least one polypeptide indicated in any one of claims 1 to 5

in animal feed;
in animal feed additives;
in the preparation of a composition for use in animal feed;
for improving the nutritional value of an animal feed;
for increasing digestible and/or soluble protein in animal feed;
for increasing the degree of hydrolysis of proteins in animal diets; and/or
for the treatment of proteins.

**9.** A method for improving the nutritional value of an animal feed, wherein at least one polypeptide indicated in any one of claims 1 to 5 is added to the feed.

**10.** An animal feed additive comprising

at least one polypeptide indicated in any one of claims 1 to 5; and
at least one fat soluble vitamin, and/or
at least one water soluble vitamin, and/or
at least one trace mineral.

**11.** The animal feed additive of claim 10, wherein the animal feed comprises one or more further enzymes, wherein the further enzymes are selected from the group comprising of amylases; phytases; xylanases; galactanases; alpha-galactosidases; proteases, phospholipases; and beta-glucanases, or any mixture thereof.

**12.** The animal feed additive of any of claims 10 to 11 comprising at least one probiotic or direct fed microbial (DFM).

**13.** An animal feed having a crude protein content of 50 to 800 g/kg and comprising at least one polypeptide indicated in any one of claims 1 to 5.

**14.** A method for the treatment of proteins, comprising the step of adding at least one polypeptide indicated in any one of claims 1 to 5 to at least one protein or protein source.

**15.** The method of claim 14, wherein corn, maize, soybean or soybean meal is included amongst the at least one protein source.

**Patentansprüche**

**1.** Verwendung eines isolierten Polypeptids mit $\alpha$-L-Galactosidaseaktivität, ausgewählt aus der Gruppe bestehend aus:

(a) einem Polypeptid mit mindestens 80%, z.B. mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, oder mindestens 99% Sequenzidentität zum reifen Polypeptid von SEQ ID NO: 2 oder SEQ ID NO: 3;
(b) einem Polypeptid, das durch ein Polynukleotid kodiert ist, das unter hohen Stringenzbedingungen oder sehr hohen Stringenzbedingungen mit (i) der das reife Polypeptid kodierenden Sequenz von SEQ ID NO: 1, oder (ii) dem Komplement voller Länge von (i) hybridisiert;
(c) einem Polypeptid, das durch ein Polynukleotid mit mindestens 80%, z.B. mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, oder mindestens 99% Sequenzidentität zur das reife Polypeptid kodierenden Sequenz von SEQ ID NO: 1 kodiert ist;

(d) einer Variante des reifen Polypeptids von SEQ ID NO: 2 oder SEQ ID NO: 3, umfassend eine Substitution, Deletion und/oder Insertion an einer oder mehreren Positionen, wobei die Polypeptide sich durch nicht mehr als 10 Aminosäuren unterscheiden; und

(e) einem Fragment des Polypeptids von (a), (b), (c) oder (d), das α-L-Galactosidaseaktivität aufweist, wobei das Fragment mindestens 90% der Aminosäuren des reifen Peptids enthält,

in Tierfutter.

2. Verwendung nach Anspruch 1, wobei das Polypeptid SEQ ID NO: 2, SEQ ID NO: 3, SEQ ID NO: 4, SEQ ID NO: 5 oder SEQ ID NO: 6 oder das reife Polypeptid von SEQ ID NO: 2, SEQ ID NO: 4, SEQ ID NO: 5 oder SEQ ID NO: 6 umfasst oder daraus besteht.

3. Verwendung nach einem beliebigen der Ansprüche 1 bis 2, wobei das reife Polypeptid Aminosäuren 20 bis 811 von SEQ ID NO: 2, SEQ ID NO: 3, SEQ ID NO: 4, SEQ ID NO: 5 oder SEQ ID NO: 6 ist.

4. Zusammensetzung, umfassend ein isoliertes Polypeptid mit α-L-Galactosidaseaktivität, ausgewählt aus der Gruppe bestehend aus:

(a) einem Polypeptid mit mindestens 80%, z.B. mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, mindestens 99% oder 100% Sequenzidentität zu SEQ ID NO: 2, SEQ ID NO: 3, SEQ ID NO: 4, SEQ ID NO: 5 oder SEQ ID NO: 6;

(b) einem Polypeptid, das durch ein Polynukleotid kodiert ist, das unter hohen Stringenzbedingungen oder sehr hohen Stringenzbedingungen mit (i) der das reife Polypeptid kodierenden Sequenz von SEQ ID NO: 1, und/oder (ii) dem komplementären Strand voller Länge von (i) hybridisiert;

(c) einem Polypeptid, das durch ein Polynukleotid mit mindestens 80%, z.B. mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98%, mindestens 99% oder 100% Sequenzidentität zur das reife Polypeptid kodierenden Sequenz von SEQ ID NO: 2, SEQ ID NO: 3, SEQ ID NO: 4, SEQ ID NO: 5 oder SEQ ID NO: 6 kodiert ist;

(d) einer Variante umfassend eine Substitution, Deletion und/oder Insertion von einer oder mehreren (einigen) Aminosäuren von SEQ ID NO: 3, wobei die Polypeptide sich durch nicht mehr als 30 Aminosäuren unterscheiden; und

(e) einem Fragment des Polypeptids von (a), (b), (c) oder (d), das α-L-Galactosidaseaktivität aufweist, wobei das Fragment mindestens 90% der Aminosäuren des reifen Peptids enthält.

5. Zusammensetzung nach Anspruch 4, wobei das Polypeptid SEQ ID NO: 2, SEQ ID NO:3, SEQ ID NO: 4, SEQ ID NO: 5 oder SEQ ID NO: 6 oder das reife Polypeptid von SEQ ID NO: 2, SEQ ID NO: 4, SEQ ID NO: 5 oder SEQ ID NO: 6 umfasst oder daraus besteht.

6. Zusammensetzung nach einem beliebigen der Ansprüche 4 oder 5, die in der Form eines Granulats oder eines Mikrogranulats ist.

7. Gesamtbrühenformulierung oder Zellkulturzusammensetzung, umfassend ein in einem beliebigen der Ansprüche 1-5 angegebenes Polypeptid.

8. Verwendung von mindestens einem in einem beliebigen der Ansprüche 1-5 angegebenen Polypeptid in Tierfutter;

in Tierfutterzusätzen;

in der Herstellung einer Zusammensetzung zur Verwendung in Tierfutter; zum Verbessern des Nährwerts eines Tierfutters;

zum Erhöhen von verdaulichem und/oder löslichem Protein in Tierfutter; zum Erhöhen des Grads an Hydrolyse von Proteinen in Tiernahrungen; und/oder

zur Behandlung von Proteinen.

9. Verfahren zum Verbessern des Nährwerts eines Tierfutters, wobei mindestens ein in einem beliebigen der Ansprüche 1-5 angegebenes Polypeptid zum Futter zugegeben wird.

10. Tierfutterzusatz, umfassend
mindestens ein in einem beliebigen der Ansprüche 1-5 angegebenes Polypeptid; und
mindestens ein fettlösliches Vitamin, und/oder
mindestens ein wasserlösliches Vitamin, und/oder
mindestens ein Spurenmineral.

11. Tierfutterzusatz nach Anspruch 10, wobei das Tierfutter ein oder mehrere weitere Enzyme umfasst, wobei die weiteren Enzyme ausgewählt sind aus der Gruppe umfassend Amylasen; Phytasen; Xylanasen; Galactanasen; alpha-Galactosidasen; Proteasen, Phospholipasen; und beta-Glucanasen oder ein beliebiges Gemisch davon.

12. Tierfutterzusatz nach einem beliebigen der Ansprüche 10 bis 11, das mindestens ein Probiotikum oder "Direct Fed Microbial" (DFM) umfasst.

13. Tierfutter mit einem Rohproteingehalt von 50 bis 800 g/kg und umfassend mindestens ein in einem beliebigen der Ansprüche 1 bis 5 angegebenes Polypeptid.

14. Verfahren zur Behandlung von Proteinen, umfassend den Schritt des Zugebens von mindestens einem in einem beliebigen der Ansprüche 1 bis 5 angegebenen Polypeptid zu mindestens einem Protein oder einer Proteinquelle.

15. Verfahren nach Anspruch 14, wobei Korn, Mais, Sojabohne oder Sojabohnenschrot in der mindestens einen Proteinquelle eingeschlossen ist.

**Revendications**

1. Utilisation d'un polypeptide isolé ayant une activité $\alpha$-L-galactosidase, choisi dans le groupe constitué par :

(a) un polypeptide présentant une identité de séquence d'au moins 80 %, p. ex., au moins 85 %, au moins 86 %, au moins 87 %, au moins 88 %, au moins 89 %, au moins 90 %, au moins 91 %, au moins 92 %, au moins 93 %, au moins 94 %, au moins 95 %, au moins 96 %, au moins 97 %, au moins 98 %, ou au moins 99 % avec le polypeptide mature de SEQ ID No: 2 ou SEQ ID No: 3 ;
(b) un polypeptide codé par un polynucléotide qui s'hybride dans des conditions de stringence élevées ou dans des conditions de stringence très élevées à (i) la séquence codant pour le polypeptide mature de SEQ ID No: 1, ou (ii) le brin complémentaire pleine longueur de (i) ;
(c) un polypeptide codé par un polynucléotide présentant une identité de séquence d'au moins 80 %, p. ex., au moins 85 %, au moins 86 %, au moins 87 %, au moins 88 %, au moins 89 %, au moins 90 %, au moins 91 %, au moins 92 %, au moins 93 %, au moins 94 %, au moins 95 %, au moins 96 %, au moins 97 %, au moins 98 %, ou au moins 99 % avec la séquence codant pour le polypeptide mature de SEQ ID No: 1 ;
(d) un variant du polypeptide mature de SEQ ID No: 2 ou SEQ ID No: 3 comprenant une substitution, une délétion, et/ou une insertion à une ou plusieurs positions, dans lequel les polypeptides ne diffèrent pas de plus de 10 acides aminés ; et
(e) un fragment de polypeptide selon (a), (b), (c), ou (d), qui a une activité $\alpha$-L-galactosidase dans lequel le fragment contient au moins 90 % des acides aminés du peptide mature,

dans un aliment pour animaux.

2. Utilisation selon la revendication 1, dans laquelle le polypeptide comprend ou est constitué par SEQ ID No: 2, SEQ ID No: 3, SEQ ID No: 4, SEQ ID No: 5 ou SEQ ID No: 6 ou le polypeptide mature de SEQ ID No: 2, SEQ ID No: 4, SEQ ID No: 5 ou SEQ ID No: 6.

3. Utilisation selon l'une quelconque des revendications 1 à 2, dans laquelle le polypeptide mature comprend les acides aminés 20 à 811 de SEQ ID No: 2, SEQ ID No: 3, SEQ ID No: 4, SEQ ID No: 5 ou SEQ ID No: 6.

4. Composition comprenant un polypeptide isolé ayant une activité $\alpha$-L-galactosidase, choisi dans le groupe constitué par :

(a) un polypeptide présentant une identité de séquence d'au moins 80 %, p. ex., au moins 85 %, au moins 86 %, au moins 87 %, au moins 88 %, au moins 89 %, au moins 90 %, au moins 91 %, au moins 92 %, au moins 93 %, au moins 94 %, au moins 95 %, au moins 96 %, au moins 97 %, au moins 98 %, au moins 99 %, ou de 100 % avec SEQ ID No: 2, SEQ ID No: 3, SEQ ID No: 4, SEQ ID No: 5 ou SEQ ID No: 6 ;

(b) un polypeptide codé par un polynucléotide qui s'hybride dans des conditions de stringence élevées ou dans des conditions de stringence très élevées à :

(i) la séquence codant pour le polypeptide mature de SEQ ID No: 1 ; et/ou
(ii) le brin complémentaire pleine longueur de (i) ;

(c) un polypeptide codé par un polynucléotide présentant une identité de séquence d'au moins 80 %, p. ex., au moins 85 %, au moins 86 %, au moins 87 %, au moins 88 %, au moins 89 %, au moins 90 %, au moins 91 %, au moins 92 %, au moins 93 %, au moins 94 %, au moins 95 %, au moins 96 %, au moins 97 %, au moins 98 %, au moins 99 %, ou de 100 % avec la séquence codant pour le polypeptide mature de SEQ ID No: 2, SEQ ID No: 3, SEQ ID No: 4, SEQ ID No: 5 ou SEQ ID No: 6 ;

(d) un variant comprenant une substitution, une délétion, et/ou une insertion d'un acide aminé ou plus (plusieurs) de SEQ ID No: 3, dans lequel les polypeptides ne diffèrent pas de plus de 30 acides aminés ; et

(e) un fragment de polypeptide selon (a), (b), (c), ou (d), qui a une activité α-L-galactosidase, dans lequel le fragment contient au moins 90 % des acides aminés du peptide mature.

5. Composition selon la revendication 4, dans laquelle le polypeptide comprend ou est constitué par SEQ ID No: 2, SEQ ID No: 3, SEQ ID No: 4, SEQ ID No: 5 ou SEQ ID No: 6 ou le polypeptide mature de SEQ ID No: 2, SEQ ID No: 4, SEQ ID No: 5 ou SEQ ID No: 6.

6. Composition selon l'une quelconque des revendications 4 à 5 qui est sous la forme d'un granulat ou d'un microgranulat.

7. Formulation de bouillon complet ou composition de culture cellulaire comprenant un polypeptide selon l'une quelconque des revendications 1 à 5.

8. Utilisation d'au moins un polypeptide selon l'une quelconque des revendications 1 à 5

dans un aliment pour animaux ;
dans des additifs pour aliment pour animaux ;
dans la préparation d'une composition destinée à être utilisée dans un aliment pour animaux ;
pour améliorer la valeur nutritionnelle d'un aliment pour animaux ;
pour accroître la protéine digestible et/ou soluble dans un aliment pour animaux ;
pour accroître le degré d'hydrolyse des protéines dans des régimes animaux ; et/ou pour traiter les protéines.

9. Méthode pour améliorer la valeur nutritionnelle d'un aliment pour animaux, dans laquelle au moins un polypeptide selon l'une quelconque des revendications 1 à 5 est ajouté à l'aliment.

10. Additif pour aliment pour animaux comprenant :

au moins un polypeptide selon l'une quelconque des revendications 1 à 5 ; et
au moins une vitamine soluble dans la graisse, et/ou
au moins une vitamine soluble dans l'eau, et/ou
au moins une substance minérale à l'état de traces.

11. Additif pour aliment pour animaux selon la revendication 10, dans lequel l'additif pour aliment pour animaux comprend une ou plusieurs enzymes supplémentaires, dans lequel les enzymes supplémentaires sont choisies dans le groupe comprenant les amylases ; les phytases ; les xylanases ; les galactanases ; les alpha-galactosidases ; les protéases, les phospholipases ; et les bêta-glucanases, ou un mélange quelconque de celles-ci.

12. Additif pour aliment pour animaux selon l'une quelconque des revendications 10 à 11, comprenant au moins un probiotique ou un microbe administré directement (DFM pour direct fed microbial).

13. Aliment pour animaux ayant une teneur en protéine brute de 50 à 800 g/kg et comprenant au moins un polypeptide selon l'une quelconque des revendications 1 à 5.

14. Méthode de traitement des protéines, comprenant l'étape d'ajout d'au moins un polypeptide selon l'une quelconque des revendications 1 à 5 à au moins une protéine ou source de protéines.

15. Méthode selon la revendication 14, dans laquelle le blé, le maïs, le soja ou la farine de soja font partie de ladite au moins une source de protéines.

GH95 product

A     B     C

Fig. 1

Xylose

Fucose

Galactose

Arabinose

GH95 vs Corn xylan

Corn xylan no enzyme

Fig. 2

Fig. 3

Fig. 4

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9517413 A
- WO 9522625 A
- US 5223409 A
- WO 9206204 A
- WO 9943835 A
- WO 9600787 A
- WO 0056900 A
- US 6011147 A
- WO 9425612 A
- WO 9533836 A
- WO 2010039889 A
- WO 0024883 A
- EP 238023 A
- US 6489127 B
- US 6506559 B
- US 6511824 B
- US 6515109 B
- WO 9015861 A
- WO 2010096673 A
- WO 0158275 A
- WO 03044049 A
- WO 03048148 A
- WO 9401459 A
- WO 02090384 A
- US 09779334 B
- WO 12570 A
- EP 13178257 A

### Non-patent literature cited in the description

- **ALLERDINGS et al.** *Phytochemistry,* 2006, vol. 67, 1276-1286
- **HANTUS et al.** *Carbohydr. Res.,* 1997, vol. 304, 11-20
- **ZABLACKIS et al.** *Science,* 1996, vol. 272, 1808-1810
- **REUHS et al.** *Planta,* 2004, vol. 219, 147-157
- **NIELSEN et al.** *Protein Engineering,* 1997, vol. 10, 1-6
- **NEEDLEMAN ; WUNSCH.** *J. Mol. Biol.,* 1970, vol. 48, 443-453
- **RICE et al.** *Trends Genet.,* 2000, vol. 16, 276-277
- **RICE et al.** *EMBOSS: The European Molecular Biology Open Software Suite,* 2000
- **SAMBROOK et al.** Molecular Cloning, A Laboratory Manual. Cold Spring Harbor, 1989
- **BOLTON ; MCCARTHY.** *Proc. Natl. Acad. Sci. USA,* 1962, vol. 48, 1390
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **NAGAE M ; TSUCHIYA A ; KATAYAMA T ; YAMAMOTO K ; WAKATSUKI S ; KATO R.** Structural basis of the catalytic reaction mechanism of novel 1,2-alpha-L-fucosidase from Bifidobacterium bifidum. *J Biol Chem.,* 22 June 2007, vol. 282 (25), 18497-509
- **REIDHAAR-OLSON ; SAUER.** *Science,* 1988, vol. 241, 53-57
- **BOWIE ; SAUER.** *Proc. Natl. Acad. Sci. USA,* vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **COOPER et al.** *EMBO J.,* 1993, vol. 12, 2575-2583
- **DAWSON et al.** *Science,* vol. 266, 776-779
- **MARTIN et al.** *J. Ind. Microbiol. Biotechnol.,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512

- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **INNIS et al.** PCR: A Guide to Methods and Application. Academic Press, 1990
- **FORD et al.** *Protein Expression and Purification,* 1991, vol. 2, 95-107
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** Useful proteins from recombinant bacteria. *Scientific American,* 1980, vol. 242, 74-94
- **ROMANOS et al.** *Yeast,* 1992, vol. 8, 423-488
- **HUE et al.** *Journal of Bacteriology,* 1995, vol. 177, 3465-3471
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol. (Praha),* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY et al.** *Appl. Environ. Microbiol.,* 1999, vol. 65, 3800-3804
- **CLEWELL.** *Microbiol. Rev.,* 1981, vol. 45, 409-436
- **HAWKSWORTH et al.** Ainsworth and Bisby's Dictionary of The Fungi. CAB International, University Press, 1995
- Biology and Activities of Yeast. Soc. App. Bacteriol. Symposium Series. 1980
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- **BECKER ; GUARENTE.** Guide to Yeast Genetics and Molecular Biology, Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- Protein Purification. VCH Publishers, 1989
- **J. SAMBROOK ; E.F. FRITSCH ; T. MANIATIS.** Molecular Cloning, A Laboratory Manual. Cold Spring Harbor, 1989
- **BACH KNUDSEN, K. E.** Carbohydrate and lignin contents of plant materials used in animal feeding. *Animal Feed Science and Technology,* 1997, vol. 67, 319-338
- **KJELDAHL.** Official Methods of Analysis. Association of Official Analytical Chemists, 1984
- subcommittee on swine nutrition, committee on animal nutrition, board of agriculture. national research council. National Academy Press, 1988, 2-6
- European Table of Energy Values for Poultry Feed-stuffs, Spelderholt centre for poultry research and extension. Grafisch bedrijf Ponsen & looijen bv
- **VAN VOEDERMIDDELEN.** *Central Veevoederbureau,* ISBN 90-72839-13-7
- **EGGERTH AH ; GAGNON BH.** The bacteroides of human feces. *J. Bacteriol.,* 1933, vol. 25, 389-413

SEQUENCE LISTING

<110> Novozymes A/S

<120> POLYPEPTIDES HAVING ALPHA-L-GALACTOSIDASE ACTIVITY AND POLYNUCLEOTIDES ENCODING SAME

<130> 12570-WO-PCT

<150> EP13178257.5
<151> 2013-07-26

<160> 6

<170> PatentIn version 3.5

<210> 1
<211> 2436
<212> DNA
<213> Bacteroides ovatus ATCC 8483

<220>
<221> CDS
<222> (1)..(2433)

<220>
<221> sig_peptide
<222> (1)..(57)

<220>
<221> mat_peptide
<222> (58)..(2433)

<400> 1

```
atg aac aga aac act ttt ttt gta tta ttc cta tta ata agc aac ctt        48
Met Asn Arg Asn Thr Phe Phe Val Leu Phe Leu Leu Ile Ser Asn Leu
            -15             -10                 -5

gtt tca gga cag gac ctg aaa ctg tgg tat agt caa cct gca caa aat        96
Val Ser Gly Gln Asp Leu Lys Leu Trp Tyr Ser Gln Pro Ala Gln Asn
        -1  1               5                   10

tgg tcg gaa gct ctc cct att ggt aac tcc cgt ctt gga gcc atg gta       144
Trp Ser Glu Ala Leu Pro Ile Gly Asn Ser Arg Leu Gly Ala Met Val
        15              20                  25

tat ggc gga atc gaa cgc gaa gaa tta caa ctc aac gaa gaa aca ttt       192
Tyr Gly Gly Ile Glu Arg Glu Glu Leu Gln Leu Asn Glu Glu Thr Phe
30                  35                  40                  45

tgg gca ggt tct cca tac aat aat aat aac ccg aat gcc gta cat gtg       240
Trp Ala Gly Ser Pro Tyr Asn Asn Asn Asn Pro Asn Ala Val His Val
                50                  55                  60

cta ccg gtt gta cgc aaa ctg att ttt gaa ggg aga aac aag gaa gcg       288
Leu Pro Val Val Arg Lys Leu Ile Phe Glu Gly Arg Asn Lys Glu Ala
                65                  70                  75

caa aga ctc att gac gcc aac ttc tta acc cag caa cac gga atg agc       336
Gln Arg Leu Ile Asp Ala Asn Phe Leu Thr Gln Gln His Gly Met Ser
                80                  85                  90
```

```
tat ctg aca tta ggt agt ctt tac ctg gaa ttt ccg gaa cac caa aac       384
Tyr Leu Thr Leu Gly Ser Leu Tyr Leu Glu Phe Pro Glu His Gln Asn
    95                  100                 105

gga tcc ggc ttt tac cgc gat ctg aat ctg gag aat gca acg aca act       432
Gly Ser Gly Phe Tyr Arg Asp Leu Asn Leu Glu Asn Ala Thr Thr Thr
110                 115                 120                 125

acc cgt tat caa gtg gat gac gtc act tat acc cgc acc act ttt gct       480
Thr Arg Tyr Gln Val Asp Asp Val Thr Tyr Thr Arg Thr Thr Phe Ala
                130                 135                 140

tca ttt act gat aat gtc att att atg cat atc aaa gca agt aaa gca       528
Ser Phe Thr Asp Asn Val Ile Ile Met His Ile Lys Ala Ser Lys Ala
                145                 150                 155

aac gca ctg aac ttt act att gca tat aac tgc cct cta gta cat aag       576
Asn Ala Leu Asn Phe Thr Ile Ala Tyr Asn Cys Pro Leu Val His Lys
            160                 165                 170

gtg aat gtt cag aac gat cag ctg acc gtc acc tgt caa ggg aaa gag       624
Val Asn Val Gln Asn Asp Gln Leu Thr Val Thr Cys Gln Gly Lys Glu
    175                 180                 185

cag gaa ggt ttg aaa gcc gca cta cgg gca gaa tgt caa atc cag gta       672
Gln Glu Gly Leu Lys Ala Ala Leu Arg Ala Glu Cys Gln Ile Gln Val
190                 195                 200                 205

aaa acg aat ggc act ctc cgc ccg gca gga aat acg ctc caa ata aat       720
Lys Thr Asn Gly Thr Leu Arg Pro Ala Gly Asn Thr Leu Gln Ile Asn
                210                 215                 220

gaa gga aca gaa gcc acc ctt tat ata tcg gca gca acc aac tac gta       768
Glu Gly Thr Glu Ala Thr Leu Tyr Ile Ser Ala Ala Thr Asn Tyr Val
                225                 230                 235

aac tac cag gat gtc agc gcc gat gaa tcc cac cga acc agc gaa tat       816
Asn Tyr Gln Asp Val Ser Ala Asp Glu Ser His Arg Thr Ser Glu Tyr
                240                 245                 250

ctg aaa aga gca atg caa ata cct tac gaa aaa gct ctc aaa aac cat       864
Leu Lys Arg Ala Met Gln Ile Pro Tyr Glu Lys Ala Leu Lys Asn His
    255                 260                 265

atc gct tat tat aaa aaa caa ttt gac cgt gta cgc ctt aca tta ccg       912
Ile Ala Tyr Tyr Lys Lys Gln Phe Asp Arg Val Arg Leu Thr Leu Pro
270                 275                 280                 285

gca ggc aaa gct tcc caa ctg gag act ccc aaa cga att gaa aac ttc       960
Ala Gly Lys Ala Ser Gln Leu Glu Thr Pro Lys Arg Ile Glu Asn Phe
                290                 295                 300

gga aat ggg gaa gac atg gca atg gca gct cta ctt ttc cat tat gga      1008
Gly Asn Gly Glu Asp Met Ala Met Ala Ala Leu Leu Phe His Tyr Gly
                305                 310                 315

cgt tat ctg ctg att tct tct tcg caa ccg ggt ggg caa ccg gct aat      1056
Arg Tyr Leu Leu Ile Ser Ser Ser Gln Pro Gly Gly Gln Pro Ala Asn
    320                 325                 330

ctg caa ggt ata tgg aat aac agt act cat gca ccc tgg gat agc aaa      1104
Leu Gln Gly Ile Trp Asn Asn Ser Thr His Ala Pro Trp Asp Ser Lys
    335                 340                 345
```

36

```
tac acc atc aat atc aat aca gaa atg aac tat tgg ccg gca gaa gtc      1152
Tyr Thr Ile Asn Ile Asn Thr Glu Met Asn Tyr Trp Pro Ala Glu Val
350             355             360             365

act aat tta agt gaa aca cat agt ccg tta ttc tct atg ttg aaa gat      1200
Thr Asn Leu Ser Glu Thr His Ser Pro Leu Phe Ser Met Leu Lys Asp
            370             375             380

tta tcc gtc aca gga gca gaa aca gcc cga acc atg tat gat tgt cgg      1248
Leu Ser Val Thr Gly Ala Glu Thr Ala Arg Thr Met Tyr Asp Cys Arg
            385             390             395

gga tgg gtg gca cat cat aac act gac tta tgg aga atc tgt ggt gta      1296
Gly Trp Val Ala His His Asn Thr Asp Leu Trp Arg Ile Cys Gly Val
            400             405             410

gtc gat ttt gca gca gcc ggc atg tgg cct agc ggc ggc gca tgg tta      1344
Val Asp Phe Ala Ala Ala Gly Met Trp Pro Ser Gly Gly Ala Trp Leu
            415             420             425

gct caa cat atc tgg caa cat tac ctc ttt aca gga aat aaa gag ttc      1392
Ala Gln His Ile Trp Gln His Tyr Leu Phe Thr Gly Asn Lys Glu Phe
430             435             440             445

ctg aaa gag tat tat ccc atc ctg aaa gga aca gcg cag ttc tac atg      1440
Leu Lys Glu Tyr Tyr Pro Ile Leu Lys Gly Thr Ala Gln Phe Tyr Met
            450             455             460

gat ttc ttg gta gaa cat cca gtg tat aaa tgg ttg gta gtg tcg cct      1488
Asp Phe Leu Val Glu His Pro Val Tyr Lys Trp Leu Val Val Ser Pro
            465             470             475

tct gtg tcg ccg gaa cac ggt ccc atc acg gcc ggt tgc aca atg gac      1536
Ser Val Ser Pro Glu His Gly Pro Ile Thr Ala Gly Cys Thr Met Asp
            480             485             490

aat cag att gcc ttt gat gca ctg cat aat acc tta ttg gct tca tac      1584
Asn Gln Ile Ala Phe Asp Ala Leu His Asn Thr Leu Leu Ala Ser Tyr
            495             500             505

atc gca ggt gaa gcc ccc tct ttc caa gac tcc ctc aaa caa aca cta      1632
Ile Ala Gly Glu Ala Pro Ser Phe Gln Asp Ser Leu Lys Gln Thr Leu
510             515             520             525

gag aag ttg cca cct atg caa ata gga aag cat aac caa cta caa gaa      1680
Glu Lys Leu Pro Pro Met Gln Ile Gly Lys His Asn Gln Leu Gln Glu
            530             535             540

tgg ctt gaa gat att gac aat ccc aag gac gag cac cgt cat att tca      1728
Trp Leu Glu Asp Ile Asp Asn Pro Lys Asp Glu His Arg His Ile Ser
            545             550             555

cat ttg tac ggg cta tat ccg agt aat caa att tct cca tac tca aat      1776
His Leu Tyr Gly Leu Tyr Pro Ser Asn Gln Ile Ser Pro Tyr Ser Asn
            560             565             570

ccc gag tta ttc cag gca gca aga aac aca ttg ctt caa cgc ggt gat      1824
Pro Glu Leu Phe Gln Ala Ala Arg Asn Thr Leu Leu Gln Arg Gly Asp
575             580             585

aag gct acc ggt tgg agt atc ggc tgg aaa gtc aat ttc tgg gca cgt      1872
Lys Ala Thr Gly Trp Ser Ile Gly Trp Lys Val Asn Phe Trp Ala Arg
```

590                          595                          600                          605

```
atg ctg gat gga aac cat gcc ttc caa atc att aaa aat atg att caa    1920
Met Leu Asp Gly Asn His Ala Phe Gln Ile Ile Lys Asn Met Ile Gln
                610             615                 620

cta ttg ccc aac gat cac ttg gct aaa gaa tat ccg aac ggg cgt acc    1968
Leu Leu Pro Asn Asp His Leu Ala Lys Glu Tyr Pro Asn Gly Arg Thr
                625             630                 635

tat ccc aat atg ctg gat gcc cat ccg cct ttc cag att gac ggt aat    2016
Tyr Pro Asn Met Leu Asp Ala His Pro Pro Phe Gln Ile Asp Gly Asn
                640             645                 650

ttc ggt tat aca gcc gga gta gcg gaa atg ttg ctc caa agt cat gac    2064
Phe Gly Tyr Thr Ala Gly Val Ala Glu Met Leu Leu Gln Ser His Asp
    655                 660                 665

ggt gcc gta cat tta ttg ccg gca ttg cct gat gca tgg gaa gaa ggg    2112
Gly Ala Val His Leu Leu Pro Ala Leu Pro Asp Ala Trp Glu Glu Gly
670                 675                 680                 685

agt gtg aag ggg ctg gtg gca cgt gga aac ttt act gtc gat atg gac    2160
Ser Val Lys Gly Leu Val Ala Arg Gly Asn Phe Thr Val Asp Met Asp
                690                 695                 700

tgg aaa aac aat gtg tta aat aaa gcg atc atc cga tcg aat ata ggc    2208
Trp Lys Asn Asn Val Leu Asn Lys Ala Ile Ile Arg Ser Asn Ile Gly
                705                 710                 715

agt acg ctc cgc atc cgg tct tat gtc cca cta aaa ggg aaa ggc tta    2256
Ser Thr Leu Arg Ile Arg Ser Tyr Val Pro Leu Lys Gly Lys Gly Leu
                720                 725                 730

aaa caa gta aac ggg aaa gag tgc tca aat aga tta ttc gcc acc act    2304
Lys Gln Val Asn Gly Lys Glu Cys Ser Asn Arg Leu Phe Ala Thr Thr
                735                 740                 745

cct atc aag caa cct ctt gta gcc aaa gga gta agt gcg cag tct ccc    2352
Pro Ile Lys Gln Pro Leu Val Ala Lys Gly Val Ser Ala Gln Ser Pro
750                 755                 760                 765

aaa ctg cag aag gtt tac gaa tat gac atc gaa aca aag gcg ggt aaa    2400
Lys Leu Gln Lys Val Tyr Glu Tyr Asp Ile Glu Thr Lys Ala Gly Lys
                770                 775                 780

acc tat att gta aat aca atc gaa gga aaa caa taa                    2436
Thr Tyr Ile Val Asn Thr Ile Glu Gly Lys Gln
                785                 790
```

<210> 2
<211> 811
<212> PRT
<213> Bacteroides ovatus ATCC 8483

<400> 2

Met Asn Arg Asn Thr Phe Phe Val Leu Phe Leu Leu Ile Ser Asn Leu
              -15                    -10                    -5

Val Ser Gly Gln Asp Leu Lys Leu Trp Tyr Ser Gln Pro Ala Gln Asn

```
                 -1  1                    5                          10

        Trp Ser Glu Ala Leu Pro Ile Gly Asn Ser Arg Leu Gly Ala Met Val
            15                  20              25

        Tyr Gly Gly Ile Glu Arg Glu Glu Leu Gln Leu Asn Glu Glu Thr Phe
        30                  35                  40                      45

        Trp Ala Gly Ser Pro Tyr Asn Asn Asn Asn Pro Asn Ala Val His Val
                        50                  55                  60

        Leu Pro Val Val Arg Lys Leu Ile Phe Glu Gly Arg Asn Lys Glu Ala
                        65                  70                  75

        Gln Arg Leu Ile Asp Ala Asn Phe Leu Thr Gln Gln His Gly Met Ser
                80                  85                  90

        Tyr Leu Thr Leu Gly Ser Leu Tyr Leu Glu Phe Pro Glu His Gln Asn
            95                  100                 105

        Gly Ser Gly Phe Tyr Arg Asp Leu Asn Leu Glu Asn Ala Thr Thr Thr
        110                 115                 120                     125

        Thr Arg Tyr Gln Val Asp Asp Val Thr Tyr Thr Arg Thr Thr Phe Ala
                        130                 135                 140

        Ser Phe Thr Asp Asn Val Ile Ile Met His Ile Lys Ala Ser Lys Ala
                145                 150                 155

        Asn Ala Leu Asn Phe Thr Ile Ala Tyr Asn Cys Pro Leu Val His Lys
                160                 165                 170

        Val Asn Val Gln Asn Asp Gln Leu Thr Val Thr Cys Gln Gly Lys Glu
                175                 180                 185

        Gln Glu Gly Leu Lys Ala Ala Leu Arg Ala Glu Cys Gln Ile Gln Val
        190                 195                 200                     205

        Lys Thr Asn Gly Thr Leu Arg Pro Ala Gly Asn Thr Leu Gln Ile Asn
                        210                 215                 220

        Glu Gly Thr Glu Ala Thr Leu Tyr Ile Ser Ala Ala Thr Asn Tyr Val
                        225                 230                 235

        Asn Tyr Gln Asp Val Ser Ala Asp Glu Ser His Arg Thr Ser Glu Tyr
                240                 245                 250
```

40

```
Leu Lys Arg Ala Met Gln Ile Pro Tyr Glu Lys Ala Leu Lys Asn His
    255                 260                 265

Ile Ala Tyr Tyr Lys Lys Gln Phe Asp Arg Val Arg Leu Thr Leu Pro
270                 275                 280                 285

Ala Gly Lys Ala Ser Gln Leu Glu Thr Pro Lys Arg Ile Glu Asn Phe
                290                 295                 300

Gly Asn Gly Glu Asp Met Ala Met Ala Ala Leu Leu Phe His Tyr Gly
                305                 310                 315

Arg Tyr Leu Leu Ile Ser Ser Ser Gln Pro Gly Gly Gln Pro Ala Asn
                320                 325                 330

Leu Gln Gly Ile Trp Asn Asn Ser Thr His Ala Pro Trp Asp Ser Lys
                335                 340                 345

Tyr Thr Ile Asn Ile Asn Thr Glu Met Asn Tyr Trp Pro Ala Glu Val
350                 355                 360                 365

Thr Asn Leu Ser Glu Thr His Ser Pro Leu Phe Ser Met Leu Lys Asp
                370                 375                 380

Leu Ser Val Thr Gly Ala Glu Thr Ala Arg Thr Met Tyr Asp Cys Arg
                385                 390                 395

Gly Trp Val Ala His His Asn Thr Asp Leu Trp Arg Ile Cys Gly Val
                400                 405                 410

Val Asp Phe Ala Ala Ala Gly Met Trp Pro Ser Gly Gly Ala Trp Leu
415                 420                 425

Ala Gln His Ile Trp Gln His Tyr Leu Phe Thr Gly Asn Lys Glu Phe
430                 435                 440                 445

Leu Lys Glu Tyr Tyr Pro Ile Leu Lys Gly Thr Ala Gln Phe Tyr Met
                450                 455                 460

Asp Phe Leu Val Glu His Pro Val Tyr Lys Trp Leu Val Val Ser Pro
                465                 470                 475

Ser Val Ser Pro Glu His Gly Pro Ile Thr Ala Gly Cys Thr Met Asp
                480                 485                 490

Asn Gln Ile Ala Phe Asp Ala Leu His Asn Thr Leu Leu Ala Ser Tyr
                495                 500                 505
```

41

Ile Ala Gly Glu Ala Pro Ser Phe Gln Asp Ser Leu Lys Gln Thr Leu
510                 515                 520                 525

Glu Lys Leu Pro Pro Met Gln Ile Gly Lys His Asn Gln Leu Gln Glu
                530                 535                 540

Trp Leu Glu Asp Ile Asp Asn Pro Lys Asp Glu His Arg His Ile Ser
                545                 550                 555

His Leu Tyr Gly Leu Tyr Pro Ser Asn Gln Ile Ser Pro Tyr Ser Asn
                560                 565                 570

Pro Glu Leu Phe Gln Ala Ala Arg Asn Thr Leu Leu Gln Arg Gly Asp
                575                 580                 585

Lys Ala Thr Gly Trp Ser Ile Gly Trp Lys Val Asn Phe Trp Ala Arg
590                 595                 600                 605

Met Leu Asp Gly Asn His Ala Phe Gln Ile Ile Lys Asn Met Ile Gln
                610                 615                 620

Leu Leu Pro Asn Asp His Leu Ala Lys Glu Tyr Pro Asn Gly Arg Thr
                625                 630                 635

Tyr Pro Asn Met Leu Asp Ala His Pro Pro Phe Gln Ile Asp Gly Asn
                640                 645                 650

Phe Gly Tyr Thr Ala Gly Val Ala Glu Met Leu Leu Gln Ser His Asp
                655                 660                 665

Gly Ala Val His Leu Leu Pro Ala Leu Pro Asp Ala Trp Glu Glu Gly
670                 675                 680                 685

Ser Val Lys Gly Leu Val Ala Arg Gly Asn Phe Thr Val Asp Met Asp
                690                 695                 700

Trp Lys Asn Asn Val Leu Asn Lys Ala Ile Ile Arg Ser Asn Ile Gly
                705                 710                 715

Ser Thr Leu Arg Ile Arg Ser Tyr Val Pro Leu Lys Gly Lys Gly Leu
                720                 725                 730

Lys Gln Val Asn Gly Lys Glu Cys Ser Asn Arg Leu Phe Ala Thr Thr
                735                 740                 745

Pro Ile Lys Gln Pro Leu Val Ala Lys Gly Val Ser Ala Gln Ser Pro
750                 755                 760                 765

```
Lys Leu Gln Lys Val Tyr Glu Tyr Asp Ile Glu Thr Lys Ala Gly Lys
                770                 775                 780

Thr Tyr Ile Val Asn Thr Ile Glu Gly Lys Gln
                785                 790
```

<210> 3
<211> 811
<212> PRT
<213> Bacteroides ovatus ATCC 8483

<220>
<221> SIGNAL
<222> (-19)..(-1)

<220>
<221> mat_peptide
<222> (1)..(792)

<400> 3

```
Met Asn Arg Asn Thr Phe Phe Val Leu Phe Leu Leu Ile Ser Asn Leu
                -15                 -10                 -5

Val Ser Gly Gln Asp Leu Lys Leu Trp Tyr Ser Gln Pro Ala Gln Asn
        -1  1                 5                   10

Trp Ser Glu Ala Leu Pro Ile Gly Asn Ser Arg Leu Gly Ala Met Val
        15                  20                  25

Tyr Gly Gly Ile Glu Arg Glu Glu Leu Gln Leu Asn Glu Glu Thr Phe
30                  35                  40                  45

Trp Ala Gly Ser Pro Tyr Asn Asn Asn Asn Pro Asn Ala Val His Val
                50                  55                  60

Leu Pro Val Val Arg Lys Leu Ile Phe Glu Gly Arg Asn Lys Glu Ala
                65                  70                  75

Gln Arg Leu Ile Asp Ala Asn Phe Leu Thr Gln Gln His Gly Met Ser
            80                  85                  90

Tyr Leu Thr Leu Gly Ser Leu Tyr Leu Glu Phe Pro Glu His Gln Asn
        95                  100                 105

Gly Ser Gly Phe Tyr Arg Asp Leu Asn Leu Glu Asn Ala Thr Thr Thr
110                 115                 120                 125

Thr Arg Tyr Gln Val Asp Asp Val Thr Tyr Thr Arg Thr Thr Phe Ala
```

|     |     |     | 130 |     |     |     |     | 135 |     |     |     |     | 140 |     |     |
|-----|-----|-----|-----|-----|-----|-----|-----|-----|-----|-----|-----|-----|-----|-----|-----|

Ser Phe Thr Asp Asn Val Ile Ile Met His Ile Lys Ala Ser Lys Ala
                145                  150            155

Asn Ala Leu Asn Phe Thr Ile Ala Tyr Asn Cys Pro Leu Val His Lys
        160              165            170

Val Asn Val Gln Asn Asp Gln Leu Thr Val Thr Cys Gln Gly Lys Glu
        175            180            185

Gln Glu Gly Leu Lys Ala Ala Leu Arg Ala Glu Cys Gln Ile Gln Val
190              195            200            205

Lys Thr Asn Gly Thr Leu Arg Pro Ala Gly Asn Thr Leu Gln Ile Asn
        210            215            220

Glu Gly Thr Glu Ala Thr Leu Tyr Ile Ser Ala Ala Thr Asn Tyr Val
        225            230            235

Asn Tyr Gln Asp Val Ser Ala Asp Glu Ser His Arg Thr Ser Glu Tyr
        240            245            250

Leu Lys Arg Ala Met Gln Ile Pro Tyr Glu Lys Ala Leu Lys Asn His
        255            260            265

Ile Ala Tyr Tyr Lys Lys Gln Phe Asp Arg Val Arg Leu Thr Leu Pro
270              275            280            285

Ala Gly Lys Ala Ser Gln Leu Glu Thr Pro Lys Arg Ile Glu Asn Phe
        290            295            300

Gly Asn Gly Glu Asp Met Ala Met Ala Ala Leu Leu Phe His Tyr Gly
        305            310            315

Arg Tyr Leu Leu Ile Ser Ser Ser Gln Pro Gly Gly Gln Pro Ala Asn
        320            325            330

Leu Gln Gly Ile Trp Asn Asn Ser Thr His Ala Pro Trp Asp Ser Lys
        335            340            345

Tyr Thr Ile Asn Ile Asn Thr Glu Met Asn Tyr Trp Pro Ala Glu Val
350              355            360            365

Thr Asn Leu Ser Glu Thr His Ser Pro Leu Phe Ser Met Leu Lys Asp
        370            375            380

```
Leu Ser Val Thr Gly Ala Glu Thr Ala Arg Thr Met Tyr Asp Cys Arg
            385                 390                 395

Gly Trp Val Ala His His Asn Thr Asp Leu Trp Arg Ile Cys Gly Val
            400                 405                 410

Val Asp Phe Ala Ala Ala Gly Met Trp Pro Ser Gly Gly Ala Trp Leu
            415                 420                 425

Ala Gln His Ile Trp Gln His Tyr Leu Phe Thr Gly Asn Lys Glu Phe
430                 435                 440                 445

Leu Lys Glu Tyr Tyr Pro Ile Leu Lys Gly Thr Ala Gln Phe Tyr Met
                450                 455                 460

Asp Phe Leu Val Glu His Pro Val Tyr Lys Trp Leu Val Val Ser Pro
            465                 470                 475

Ser Val Ser Pro Glu His Gly Pro Ile Thr Ala Gly Cys Thr Met Asp
            480                 485                 490

Asn Gln Ile Ala Phe Asp Ala Leu His Asn Thr Leu Leu Ala Ser Tyr
            495                 500                 505

Ile Ala Gly Glu Ala Pro Ser Phe Gln Asp Ser Leu Lys Gln Thr Leu
510                 515                 520                 525

Glu Lys Leu Pro Pro Met Gln Ile Gly Lys His Asn Gln Leu Gln Glu
                530                 535                 540

Trp Leu Glu Asp Ile Asp Asn Pro Lys Asp Glu His Arg His Ile Ser
                545                 550                 555

His Leu Tyr Gly Leu Tyr Pro Ser Asn Gln Ile Ser Pro Tyr Ser Asn
                560                 565                 570

Pro Glu Leu Phe Gln Ala Ala Arg Asn Thr Leu Leu Gln Arg Gly Asp
            575                 580                 585

Lys Ala Thr Gly Trp Ser Ile Gly Trp Lys Val Asn Phe Trp Ala Arg
590                 595                 600                 605

Met Leu Asp Gly Asn His Ala Phe Gln Ile Ile Lys Asn Met Ile Gln
                610                 615                 620

Leu Leu Pro Asn Asp His Leu Ala Lys Glu Tyr Pro Asn Gly Arg Thr
                625                 630                 635
```

```
Tyr Pro Asn Met Leu Asp Ala His Pro Pro Phe Gln Ile Asp Gly Asn
        640             645                 650

Phe Gly Tyr Thr Ala Gly Val Ala Glu Met Leu Leu Gln Ser His Asp
        655             660                 665

Gly Ala Val His Leu Leu Pro Ala Leu Pro Asp Ala Trp Glu Glu Gly
670             675                 680                     685

Ser Val Lys Gly Leu Val Ala Arg Gly Asn Phe Thr Val Asp Met Asp
                690             695                 700

Trp Lys Asn Asn Val Leu Asn Lys Ala Ile Ile Arg Ser Asn Ile Gly
            705             710                 715

Ser Thr Leu Arg Ile Arg Ser Tyr Val Pro Leu Lys Gly Lys Gly Leu
        720             725                 730

Lys Gln Val Asn Gly Lys Glu Cys Ser Asn Arg Leu Phe Ala Thr Thr
        735             740                 745

Pro Ile Lys Gln Pro Leu Val Ala Lys Gly Val Ser Ala Gln Ser Pro
750             755                 760                     765

Lys Leu Gln Lys Val Tyr Glu Tyr Asp Ile Glu Thr Lys Ala Gly Lys
                770             775                 780

Thr Tyr Ile Val Asn Thr Ile Glu Gly Lys Gln
                785             790
```

<210> 4
<211> 811
<212> PRT
<213> Bacteroides ovatus CL02T12C04

<220>
<221> SIGNAL
<222> (-19)..(-1)

<220>
<221> mat_peptide
<222> (1)..(792)

<400> 4

```
Met Asn Arg Asn Thr Phe Phe Val Leu Phe Leu Leu Ile Ser Asn Leu
                -15             -10                 -5

Val Ser Gly Gln Asp Leu Lys Leu Trp Tyr Ser Gln Pro Ala Gln Asn
        -1  1               5                   10
```

```
Trp Ser Glu Ala Leu Pro Ile Gly Asn Ser Arg Leu Gly Ala Met Val
    15              20              25

Tyr Gly Gly Ile Glu Arg Glu Glu Leu Gln Leu Asn Glu Glu Thr Phe
30              35              40                      45

Trp Ala Gly Ser Pro Tyr Asn Asn Asn Asn Pro Asn Ala Val His Val
            50              55                      60

Leu Pro Val Val Arg Lys Leu Ile Phe Glu Gly Arg Asn Lys Glu Ala
            65              70              75

Gln Arg Leu Ile Asp Ala Asn Phe Leu Thr Gln Gln His Gly Met Ser
        80              85              90

Tyr Leu Thr Leu Gly Ser Leu Tyr Leu Glu Phe Pro Glu His Gln Asn
    95              100             105

Gly Ser Gly Phe Tyr Arg Glu Leu Asn Leu Glu Asn Ala Thr Thr Thr
110             115             120                     125

Thr Arg Tyr Gln Val Asp Asp Val Thr Tyr Thr Arg Thr Thr Phe Ala
            130             135             140

Ser Phe Thr Asp Asn Val Ile Ile Met His Ile Lys Ala Ser Lys Ala
        145             150             155

Asn Ala Leu Asn Phe Thr Ile Ala Tyr Asn Cys Pro Leu Val His Lys
        160             165             170

Val Asn Val Gln Asn Asp Gln Leu Thr Val Thr Cys Gln Gly Lys Glu
    175             180             185

Gln Glu Gly Leu Lys Ala Ala Leu Arg Ala Glu Cys Gln Ile Gln Val
190             195             200                     205

Lys Thr Asn Gly Thr Leu Arg Pro Ala Gly Asn Thr Leu Gln Ile Asn
            210             215                     220

Glu Gly Thr Glu Ala Thr Leu Tyr Ile Ser Ala Ala Thr Asn Tyr Val
            225             230             235

Asn Tyr Gln Asp Val Ser Ala Asp Glu Ser His Arg Thr Ser Glu Tyr
        240             245             250

Leu Lys Arg Ala Met Gln Ile Pro Tyr Glu Lys Ala Leu Lys Asn His
```

```
                255                  260                  265


Ile Ala Tyr Tyr Lys Lys Gln Phe Asp Arg Val Arg Leu Thr Leu Pro
270              275              280              285


Ala Gly Lys Ala Ser Gln Leu Glu Thr Pro Lys Arg Ile Glu Asn Phe
                290              295              300


Gly Asn Gly Glu Asp Met Ala Met Ala Ala Leu Leu Phe His Tyr Gly
                305              310              315


Arg Tyr Leu Leu Ile Ser Ser Ser Gln Pro Gly Gly Gln Pro Ala Asn
        320              325              330


Leu Gln Gly Ile Trp Asn Asn Ser Thr His Ala Pro Trp Asp Ser Lys
        335              340              345


Tyr Thr Ile Asn Ile Asn Thr Glu Met Asn Tyr Trp Pro Ala Glu Val
350              355              360              365


Thr Asn Leu Ser Glu Thr His Ser Pro Leu Phe Ser Met Leu Lys Asp
                370              375              380


Leu Ser Val Thr Gly Ala Glu Thr Ala Arg Thr Met Tyr Asp Cys Arg
        385              390              395


Gly Trp Val Ala His His Asn Thr Asp Leu Trp Arg Ile Cys Gly Val
        400              405              410


Val Asp Phe Ala Ala Ala Gly Met Trp Pro Ser Gly Gly Ala Trp Leu
        415              420              425


Ala Gln His Ile Trp Gln His Tyr Leu Phe Thr Gly Asn Lys Glu Phe
430              435              440              445


Leu Lys Glu Tyr Tyr Pro Ile Leu Lys Gly Thr Ala Gln Phe Tyr Met
                450              455              460


Asp Phe Leu Val Glu His Pro Val Tyr Lys Trp Leu Val Val Ser Pro
        465              470              475


Ser Val Ser Pro Glu His Gly Pro Ile Thr Ala Gly Cys Thr Met Asp
        480              485              490


Asn Gln Ile Ala Phe Asp Ala Leu His Asn Thr Leu Leu Ala Ser Tyr
        495              500              505
```

```
Ile Ala Gly Glu Ala Pro Ser Phe Gln Asp Ser Leu Lys Gln Thr Leu
510                 515             520                 525
2

Glu Lys Leu Pro Pro Met Gln Ile Gly Lys His Asn Gln Leu Gln Glu
                530             535                 540

Trp Leu Glu Asp Ile Asp Asn Pro Lys Asp Glu His Arg His Ile Ser
            545             550             555

His Leu Tyr Gly Leu Tyr Pro Ser Asn Gln Ile Ser Pro Tyr Ser Asn
        560             565             570

Pro Glu Leu Phe Gln Ala Ala Arg Asn Thr Leu Leu Gln Arg Gly Asp
    575             580             585

Lys Ala Thr Gly Trp Ser Ile Gly Trp Lys Val Asn Phe Trp Ala Arg
590             595             600             605

Met Leu Asp Gly Asn His Ala Phe Gln Ile Ile Lys Asn Met Ile Gln
            610             615             620

Leu Leu Pro Asn Asp His Leu Ala Lys Glu Tyr Pro Asn Gly Arg Thr
        625             630             635

Tyr Pro Asn Met Leu Asp Ala His Pro Pro Phe Gln Ile Asp Gly Asn
    640             645             650

Phe Gly Tyr Thr Ala Gly Val Ala Glu Met Leu Leu Gln Ser His Asp
    655             660             665

Gly Ala Val His Leu Leu Pro Ala Leu Pro Asp Ala Trp Glu Glu Gly
670             675             680             685

Ser Val Lys Gly Leu Val Ala Arg Gly Asn Phe Thr Val Asp Met Asp
            690             695             700

Trp Lys Asn Asn Val Leu Asn Lys Ala Ile Ile Arg Ser Asn Ile Gly
        705             710             715

Ser Thr Leu Arg Ile Arg Ser Tyr Val Pro Leu Lys Gly Lys Gly Leu
    720             725             730

Lys Gln Val Asn Gly Lys Glu Cys Ser Asn Arg Leu Phe Ala Thr Thr
    735             740             745

Pro Ile Lys Gln Pro Leu Val Ala Lys Gly Val Ser Ala Gln Ser Pro
750             755             760             765
```

49

```
        Lys Leu Gln Lys Val Tyr Glu Tyr Asp Ile Glu Thr Lys Ala Gly Lys
                        770                 775                 780

        Thr Tyr Ile Val Asn Thr Ile Glu Gly Lys Gln
                        785                 790
```

<210> 5
<211> 811
<212> PRT
<213> Bacteroides xylanisolvens CL03T12C04

<220>
<221> SIGNAL
<222> (-19)..(-1)

<220>
<221> mat_peptide
<222> (1)..(792)

<400> 5

```
        Met Asn Arg Asn Thr Phe Phe Val Leu Phe Leu Leu Ile Ser Asn Leu
                        -15                 -10                 -5

        Val Ser Gly Gln Asp Leu Lys Leu Trp Tyr Ser Gln Pro Ala Gln Asn
                        -1  1               5                   10

        Trp Ser Glu Ala Leu Pro Ile Gly Asn Ser Arg Leu Gly Ala Met Val
                        15                  20                  25

        Tyr Gly Gly Ile Glu Arg Glu Glu Leu Gln Leu Asn Glu Glu Thr Phe
        30                  35                  40                  45

        Trp Ala Gly Ser Pro Tyr Asn Asn Asn Asn Pro Asn Ala Val His Val
                        50                  55                  60

        Leu Pro Ile Val Arg Lys Leu Ile Phe Glu Gly Arg Asn Lys Glu Ala
                        65                  70                  75

        Gln Arg Leu Ile Asp Ala Asn Phe Leu Thr Gln Gln His Gly Met Ser
                        80                  85                  90

        Tyr Leu Thr Leu Gly Ser Leu Tyr Leu Glu Phe Pro Glu His Gln Asn
                        95                  100                 105

        Ala Ser Gly Phe Tyr Arg Asp Leu Asn Leu Glu Asn Ala Thr Thr Thr
                        110                 115                 120                 125

        Thr Arg Tyr Gln Val Asp Asp Val Thr Tyr Thr Arg Thr Thr Phe Ala
                        130                 135                 140
```

```
Ser Phe Thr Asp Asn Val Ile Ile Met His Ile Lys Ala Ser Lys Ala
        145                 150                 155

Asn Ala Leu Asn Phe Thr Ile Ala Tyr Asn Cys Pro Leu Val His Lys
        160                 165                 170

Val Asn Val Gln Asn Asp Gln Leu Thr Val Thr Cys Gln Gly Lys Glu
        175                 180                 185

Gln Glu Gly Leu Lys Ala Ala Leu Arg Ala Glu Cys Gln Ile Gln Val
190                 195                 200                 205

Lys Thr Asn Gly Thr Leu Arg Pro Ala Gly Asn Thr Leu Gln Ile Asn
                210                 215                 220

Glu Gly Thr Glu Ala Thr Leu Tyr Ile Ser Ala Ala Thr Asn Tyr Val
                225                 230                 235

Asn Tyr Gln Asp Val Ser Ala Asp Glu Ser Arg Arg Thr Ser Glu Tyr
        240                 245                 250

Leu Lys Arg Ala Met Gln Ile Pro Tyr Glu Lys Ala Leu Lys Ser His
        255                 260                 265

Ile Ala Tyr Tyr Lys Lys Gln Phe Asp Arg Val Arg Leu Thr Leu Pro
270                 275                 280                 285

Thr Gly Lys Thr Ser Gln Leu Glu Thr Pro Lys Arg Ile Glu Asn Phe
                290                 295                 300

Gly Asn Gly Glu Asp Met Ala Met Ala Ala Leu Leu Phe His Tyr Gly
                305                 310                 315

Arg Tyr Leu Leu Ile Ser Ser Ser Gln Pro Gly Gly Gln Pro Ala Asn
        320                 325                 330

Leu Gln Gly Ile Trp Asn Asn Ser Thr His Ala Pro Trp Asp Ser Lys
        335                 340                 345

Tyr Thr Ile Asn Ile Asn Thr Glu Met Asn Tyr Trp Pro Ala Glu Val
350                 355                 360                 365

Thr Asn Leu Ser Glu Thr His Ser Pro Leu Phe Ser Met Leu Lys Asp
                370                 375                 380

Leu Ser Val Thr Gly Ala Glu Thr Ala Arg Thr Met Tyr Asp Cys Arg
```

```
                    385                         390                         395


        Gly Trp Met Ala His His Asn Thr Asp Leu Trp Arg Ile Cys Gly Val
                400                     405                 410


        Val Asp Phe Ala Ala Ala Gly Met Trp Pro Ser Gly Gly Ala Trp Leu
            415                 420                 425


        Ala Gln His Ile Trp Gln His Tyr Leu Phe Thr Gly Asn Lys Glu Phe
        430                 435                 440                     445


        Leu Lys Glu Tyr Tyr Pro Ile Leu Lys Gly Thr Ala Gln Phe Tyr Met
                        450                 455                 460


        Asp Phe Leu Val Glu His Pro Val Tyr Lys Trp Leu Val Val Ser Pro
                    465                 470                 475


        Ser Val Ser Pro Glu His Gly Pro Ile Thr Ala Gly Cys Thr Met Asp
                480                 485                 490


        Asn Gln Ile Ala Phe Asp Ala Leu His Asn Thr Leu Leu Ala Ser Tyr
                495                 500                 505


        Ile Ala Gly Glu Ala Pro Ser Phe Gln Asp Ser Leu Lys Gln Thr Leu
        510                 515                 520                     525


        Glu Lys Leu Pro Pro Met Gln Ile Gly Lys His Asn Gln Leu Gln Glu
                        530                 535                 540


        Trp Leu Glu Asp Ile Asp Asn Pro Lys Asp Glu His Arg His Ile Ser
                    545                 550                 555


        His Leu Tyr Gly Leu Tyr Pro Ser Asn Gln Ile Ser Pro Tyr Ser Asn
                560                 565                 570


        Pro Glu Leu Phe Gln Ala Ala Arg Asn Thr Leu Leu Gln Arg Gly Asp
                575                 580                 585


        Lys Ala Thr Gly Trp Ser Ile Gly Trp Lys Val Asn Phe Trp Ala Arg
        590                 595                 600                     605


        Met Leu Asp Gly Asn His Ala Phe Gln Ile Ile Lys Asn Met Ile Gln
                        610                 615                 620


        Leu Leu Pro Asn Asp His Leu Ala Lys Glu Tyr Pro Asn Gly Arg Thr
                        625                 630                 635
```

```
Tyr Pro Asn Met Leu Asp Ala His Pro Pro Phe Gln Ile Asp Gly Asn
        640                 645                 650

Phe Gly Tyr Thr Ala Gly Val Ala Glu Met Leu Leu Gln Ser His Asp
        655                 660                 665

Gly Ala Val His Leu Leu Pro Ala Leu Pro Asp Ala Trp Glu Glu Gly
670                 675                 680                 685

Ser Val Lys Gly Leu Val Ala Arg Gly Asn Phe Thr Val Asp Met Asp
                690                 695                 700

Trp Lys Asn Asn Val Leu Asn Lys Ala Ile Ile Arg Ser Asn Ile Gly
                705                 710                 715

Gly Thr Leu Arg Ile Arg Ser Tyr Val Pro Leu Lys Gly Lys Gly Leu
        720                 725                 730

Lys Gln Val Asn Gly Lys Glu Cys Ser Asn Arg Leu Phe Ala Thr Thr
        735                 740                 745

Pro Ile Lys Arg Pro Leu Val Ala Lys Gly Val Ser Ala Gln Ser Pro
750                 755                 760                 765

Lys Leu Gln Lys Val Tyr Glu Tyr Asp Ile Glu Thr Lys Ala Gly Lys
                770                 775                 780

Thr Tyr Ile Val Asn Thr Ile Glu Gly Lys Gln
        785                 790
```

<210> 6
<211> 811
<212> PRT
<213> Bacteroides sp. 2_1_22

<220>
<221> SIGNAL
<222> (-19)..(-1)

<220>
<221> mat_peptide
<222> (1)..(792)

<400> 6

```
Met Asn Arg Asn Thr Leu Phe Leu Leu Leu Leu Leu Thr Ser Asn Leu
                -15                 -10                 -5

Val Ser Gly Gln Asp Leu Lys Leu Trp Tyr Ser Gln Pro Ala Lys Asn
        -1  1                 5                   10
```

```
Trp Ser Glu Ala Leu Pro Ile Gly Asn Ser Arg Leu Gly Ala Met Val
    15              20              25

Tyr Gly Gly Thr Glu Arg Glu Glu Leu Gln Leu Asn Glu Glu Thr Phe
30              35              40              45

Trp Ala Gly Ser Pro Tyr Asn Asn Asn Asn Pro Asn Ala Val His Val
            50              55              60

Leu Pro Ile Val Arg Lys Leu Ile Phe Glu Gly Arg Asn Lys Glu Ala
            65              70              75

Gln Arg Leu Ile Asp Ala Asn Phe Leu Thr Arg Gln His Gly Met Ser
        80              85              90

Tyr Leu Thr Leu Gly Asn Leu Tyr Leu Glu Phe Pro Gly His Lys Asp
    95              100             105

Ala Asp Asp Phe Tyr Arg Asp Leu Asn Leu Glu Asn Ala Thr Thr Thr
110             115             120             125

Thr Arg Tyr Gln Val Asn Gly Ile Asn Tyr Thr Arg Thr Thr Phe Ala
            130             135             140

Ser Phe Thr Asp Asn Val Ile Ile Met His Ile Lys Ala Ser Gln Pro
            145             150             155

Asn Ala Leu Asn Phe Asn Val Ser Tyr Asn Cys Pro Leu Lys Asn Glu
        160             165             170

Val Asn Val Gln Asn Asp Lys Leu Ile Ile Thr Cys Gln Gly Lys Glu
    175             180             185

Gln Glu Gly Met Lys Ala Ala Leu Arg Ala Glu Cys Gln Val Gln Val
190             195             200             205

Lys Thr Asp Gly Ile Ile His Pro Ala Gly Asn Ile Leu Gln Ile Asn
            210             215             220

Gly Gly Thr Glu Ala Thr Leu Tyr Ile Ser Ala Ala Thr Asn Tyr Val
            225             230             235

Asn Tyr Gln Asn Val Ser Ala Asp Glu Ser Arg Arg Thr Thr Asp Tyr
        240             245             250

Leu Glu Glu Ala Ile Leu Ile Pro Tyr Glu Lys Ala Leu Lys Glu His
    255             260             265
```

54

```
Ile Ala Phe Tyr Lys Lys Gln Phe Asp Arg Val Gln Leu His Leu Pro
270             275             280                 285

Ser Ser Glu Ala Ser Gln Ile Glu Thr Pro Arg Arg Ile Glu Asn Phe
                290             295                 300

Gly Gln Gly Asn Asp Met Ala Met Ala Ala Leu Leu Phe Gln Tyr Gly
            305             310             315

Arg Tyr Leu Leu Ile Ser Ser Ser Gln Pro Gly Gly Gln Pro Ala Asn
        320             325             330

Leu Gln Gly Ile Trp Asn Asn Ser Thr His Ala Pro Trp Asp Ser Lys
        335             340             345

Tyr Thr Ile Asn Ile Asn Thr Glu Met Asn Tyr Trp Pro Ala Glu Val
350             355             360                 365

Thr Asn Leu Ser Glu Thr His Ser Pro Leu Phe Ser Met Leu Lys Asp
            370             375             380

Leu Ser Val Thr Gly Ala Glu Thr Ala Arg Thr Met Tyr Asp Cys Trp
            385             390             395

Gly Trp Val Ala His His Asn Thr Asp Leu Trp Arg Ile Cys Gly Val
        400             405             410

Val Asp Phe Ala Ala Ala Gly Met Trp Pro Ser Gly Gly Ala Trp Leu
        415             420             425

Ala Gln His Ile Trp Gln His Tyr Leu Phe Thr Gly Asn Lys Glu Phe
430             435             440                 445

Leu Lys Glu Tyr Tyr Pro Ile Leu Lys Gly Thr Ala Gln Phe Tyr Met
                450             455             460

Asp Phe Leu Val Glu His Pro Thr Tyr Lys Trp Leu Val Val Ser Pro
            465             470             475

Ser Val Ser Pro Glu His Gly Pro Ile Thr Ala Gly Cys Thr Met Asp
            480             485             490

Asn Gln Ile Ala Phe Asp Ala Leu His Asn Thr Leu Leu Ala Ser Tyr
        495             500             505

Ile Ala Gly Glu Ala Pro Ser Phe Gln Asp Ser Leu Lys Gln Thr Leu
```

```
              510                    515                    520                    525


              Glu Lys Leu Pro Pro Met Gln Ile Gly Lys His Asn Gln Leu Gln Glu
                          530                535                540


              Trp Leu Glu Asp Ile Asp Asn Pro Lys Asp Glu His Arg His Ile Ser
                          545                550                555


              His Leu Tyr Gly Leu Tyr Pro Ser Asn Gln Ile Ser Pro Tyr Ser Asn
                          560                565                570


              Pro Glu Leu Phe Gln Ala Ala Arg Asn Thr Leu Leu Gln Arg Gly Asp
                          575                580                585


              Lys Ala Thr Gly Trp Ser Ile Gly Trp Lys Val Asn Phe Trp Ala Arg
              590                595                600                605


              Met Leu Asp Gly Asn His Ala Phe Gln Ile Ile Lys Asn Met Ile Gln
                          610                615                620


              Leu Leu Pro Asn Asp His Leu Ala Lys Glu Tyr Pro Asn Gly Arg Thr
                          625                630                635


              Tyr Pro Asn Met Leu Asp Ala His Pro Pro Phe Gln Ile Asp Gly Asn
                          640                645                650


              Phe Gly Tyr Thr Ala Gly Val Ala Glu Met Leu Leu Gln Ser His Asp
                          655                660                665


              Gly Ala Val His Leu Leu Pro Ala Leu Pro Asp Ala Trp Glu Glu Gly
              670                675                680                685


              Ser Val Lys Gly Leu Val Ala Arg Gly Asn Phe Thr Val Asp Met Asp
                          690                695                700


              Trp Lys Asn Asn Val Leu Asn Lys Ala Ile Ile Arg Ser Asn Ile Gly
                          705                710                715


              Gly Thr Leu Arg Ile Arg Ser Tyr Val Pro Leu Lys Gly Lys Gly Leu
                          720                725                730


              Lys Gln Val Asn Gly Lys Glu Cys Ser Asn Arg Leu Phe Ala Thr Thr
                          735                740                745


              Pro Ile Lys Arg Pro Leu Val Ala Lys Gly Ile Ser Ala Gln Ser Pro
              750                755                760                765
```

56

```
Lys Leu Gln Lys Val Tyr Glu Tyr Asp Ile Glu Thr Lys Ala Gly Lys
                770                 775                 780


Thr Tyr Ile Val Asn Thr Ile Glu Gly Lys Gln
                785                 790
```